# EUROPEAN PATENT SPECIFICATION

(11) **EP 4 219 439 B1**
(45) Date of publication and mention of the grant of the patent: **10.04.2024**
(21) Application number: 23150103.2
(22) Date of filing: 29.01.2020
(51) Int. Cl.: C07C 51/235, C07C 55/06, C07C 55/12, C07C 59/10, C07C 59/105, C07C 59/285

(54) **PROCESSES FOR PREPARING ALDARIC, ALDONIC, AND URONIC ACIDS**
VERFAHREN ZUR HERSTELLUNG VON ALDAR-, ALDON- UND URONSÄUREN
PROCÉDÉS DE PRÉPARATION D'ACIDES ALDARIQUES, ALDONIQUES ET URONIQUES

(30) Priority: 18.11.2019 US 201962936861 P
(43) Date of publication of application: 02.08.2023
(62) Divisional of application: 20154275.0
(73) Proprietor: Archer Daniels Midland Company, Decatur, IL 62526 (US)
(72) Inventor: Albrecht, Karl O., Decatur, 62526 (US); Brazdil, James F., Decatur, 62526 (US)
(74) Representative: dompatent von Kreisler Selting Werner - Partnerschaft von Patent- und Rechtsanwälten mbB

(56) References cited:
- US-B2- 9 770 705
- LEE ET AL.: "Pt catalysts for efficient aerobic oxidation of glucose to glucaric acid in water", GREEN CHEMISTRY, vol. 18, no. 13, 2016, pages 3815-3822, XP002800140,

## Description

### FIELD OF THE INVENTION

The invention relates to processes for preparing aldaric acids and/or lactone(s) thereof. For example, processes for preparing a C₂-C₇ aldaric acid and/or lactone(s) thereof by the catalytic oxidation of a C₂-C₇ aldonic acid and/or lactone(s) thereof and/or a C₂-C₇ aldose are described.

### BACKGROUND

For many years, there has been an interest in using biorenewable materials as a feedstock to produce commercially useful chemicals. In particular, a significant body of work has focused on the oxidation of sugars obtained from these materials to aldaric acids, especially glucose to glucaric acid. For example, see U.S. Patent Nos. 8,669,397 and 8,785,683. Not only are these products useful as intermediates in the production of monomers such as adipic acid, but also in the production of compositions having commercial applications such as de-icing fluids, acidulants, detergent builders, pH regulators, chelants, de-scalers, corrosion inhibitors, metal cleaning and finishing agents, and components of cement formulations.

Numerous catalysts and reaction conditions have been attempted for these oxidation processes. However, sustained high yields of glucaric acid over prolonged operation or at high reactor throughputs have not been demonstrated. For example, in Lee et al., "Pt catalysts for efficient aerobic oxidation of glucose to glucaric acid in water" Green Chemistry 2016, 18 (13), 3815-3822 a glucaric acid yield of 74% is reported for the oxidation of a 5 wt.% glucose feed over a 5 wt.% Pt/C catalyst at 80°C and about 1.38 MPa (200 psi) O₂ partial pressure in a batch reactor after 10 h of reaction. In U.S. Patent No. 9,770,705, a glucaric acid yield of 70% is reported for an experiment where 2.3 mL of 10 wt.% gluconic acid feed was contacted with 75 mg of catalyst at 112-126°C for 2-5 h. There remains a need for efficient and cost-effective processes for preparing aldaric acids, such as glucaric acid, and intermediates thereof from biorenewable materials that exhibit improved and stable yields, especially over prolonged operation and high reactor throughput.
US 9,770,705 B2 discloses the oxidation of glucose to glucaric acid catalysed by gold and platinum metal particles impregnated on a titanium support.

### BRIEF SUMMARY

Various aspects of the present invention are directed to processes for preparing an aldaric acid (e.g., glucaric acid) and/or lactone(s) thereof.
The present invention relates to a process for preparing an aldaric acid and/or lactone(s) thereof as defined in the appended claims.

Other objects and features will be in part apparent and in part pointed out hereinafter.

### BRIEF DESCRIPTION OF THE DRAWINGS

Figure 1 depicts a diagram showing steps of an oxidation process for preparing a C₂-C₇ aldaric acid and/or lactone(s) thereof from a C₂-C₇ aldose.
Figure 2 depicts a schematic of two-stage reaction zone.
Figure 3 depicts a schematic of two-stage reaction zone comprising different catalyst mixtures.
Figure 4 reports glucose conversion, gluconic acid yield, and glucaric acid yield in an oxidation reaction using a 1 wt.% Au/TiO₂ catalyst from 16 to 1792 hours on stream.
Figure 5 reports glucose conversion, gluconic acid yield, and glucaric acid yield in an oxidation reaction using a 1 wt.% Au/TiO₂ catalyst from 2556 to 4640 hours on stream.
Figure 6 reports the gluconic acid conversion, selectivity, and yields of glucaric acid, guluronic acid, 2-keto-gluconate, and 5-keto-gluconate achieved during an oxidation reaction at varying temperatures using a 4 wt.% Pt/C catalyst.
Figure 7 reports the glucose conversion, on-path yield, and yields of glucaric acid, gluconic acid, 2-keto-gluconate, and 5-keto-gluconate during the oxidation of glucose using a Pt-Au/TiO₂ catalyst. The pressure value given in psig is equivalent to the corresponding value in kPa following the relationship 1 psig= 108.22 KPa.
Figure 8 reports the gluconic acid conversion and yields of glucaric acid, guluronic acid, 2-keto-gluconate, and 5-keto gluconate during the oxidation of gluconic acid using a physical mixture of 4 wt.% Pt/C and 1 wt.% Au/TiO₂ catalysts. The pressure value given in psig is equivalent to the corresponding value in kPa following the relationship 1 psig= 108.22 KPa.
Figure 9 reports the gluconic acid conversion and yields of glucaric acid, guluronic acid, 2-keto-gluconate, and 5-keto-gluconate during the oxidation of gluconic acid using a staged reactor bed comprising a physical mixture of catalysts comprising Pt/C and Au/TiO₂. The pressure value given in psig is equivalent to the corresponding value in kPa following the relationship 1 psig= 108.22 KPa.
Figure 10 reports the gluconic acid conversion, selectivity, and yields of glucaric acid, guluronic acid, 2-keto-gluconate, and 5-keto-gluconate during the oxidation of gluconic acid using a physical mixture of 4 wt.% Pt/C and 1 wt.% Au/TiO2 catalysts.
Figure 11 reports the results of the experiment of Figure 10 with a total on stream time of 1468 hours.
Figure 12 reports the arabinose conversion, arabinonic acid yield, and arabinaric acid yield measured in Example 7.
Figure 13 reports the arabinonic acid conversion, arabinaric acid yield, tartaric acid yield, and glyceric acid yield measured in Example 8.
Figure 14 reports the glyceric acid yield measured in Example 9.
Figure 15 reports the glycoaldehyde conversion and glycolic acid yield measured in Example 11.

### DETAILED DESCRIPTION

Embodiments of the present invention relate to various processes for preparing aldaric acids, and/or lactone(s) of any of these acids. For example, various embodiments relate to processes for preparing a C₂-C₇ aldaric acid and/or lactone(s) thereof (e.g., glucaric acid) by the catalytic oxidation of a C₂-C₇ aldonic acid and/or lactone(s) thereof (e.g., gluconic acid). Further embodiments are directed to preparing these various acids and/or lactone(s) thereof from aldoses (e.g., glucose).

Aldoses, as referred to herein, include various compounds possessing an aldehyde and hydroxyl groups, which can be represented by formula (I):

HOCH₂(HCOH)_{w}CHO (I)

where w can be, for example, as in the invention as claimed an integer from 0 to 10 or, in some embodiments, from 0 to 5.
In various embodiments, the aldose comprises at least one C₂-C₇ aldose. In some embodiments, the aldose comprises at least one triose, tetrose, pentose, hexose, and/or heptose. Specific C₂-C₇ aldoses include, for example, glycolaldehyde, glyceraldehyde, threose, erythrose, xylose, ribose, arabinose, glucose, galactose, mannose, glucoheptose, and L-glycero-D-manno-heptose. In various embodiments, the aldose comprises a hexose such as glucose. In some embodiments, the aldose comprises a pentose such as xylose, ribose, and/or arabinose. The term "aldoses" and any specific aldose mentioned herein and as defined by formula (I) also include cyclic forms (hemiacetal forms) of these compounds.

Aldoses can be obtained from various carbohydrate-containing sources including conventional biorenewable sources such as corn grain (maize), wheat, potato, cassava and rice, as well as alternative sources such as energy crops, plant biomass, agricultural wastes, forestry residues, sugar processing residues, and plant-derived household wastes. In various embodiments, the aldose (e.g., glucose) is obtained from a grain crop (e.g., corn, wheat, soybean, rice, barley, rye, millet, sorghum, etc.). More generally, biorenewable sources that can be used include any renewable organic matter that includes a source of carbohydrates such as, for example, switch grass, miscanthus, trees (hardwood and softwood), vegetation, and crop residues (e.g., bagasse and corn stover). Other sources include, for example, waste materials (e.g., spent paper, green waste, municipal waste, etc.). Carbohydrates can be isolated from biorenewable materials using known methods.

Aldonic acids, as referred to herein, include monocarboxylic acids of formula (II):

HOCH₂(HCOH)ₓCOOH (II)

where x can be, for example, as in the invention as claimed an integer from 0 to 10 or, in some embodiments, from 0 to 5. In various embodiments, the aldonic acid comprises at least one C₂-C₇ aldonic acid. In some embodiments, the aldonic acid comprises at least one dionic acid, trionic acid, tetronic acid, pentonic acid, hexonic acid, and/or heptonic acid. Specific C₂-C₇ aldonic acids include, for example, glycolic acid, glyceric acid, threonic acid, erythronic acid, xylonic acid, ribonic acid, arabinonic acid, lyxonic acid, allonic acid, altronic acid, mannonic acid, gulonic acid, idonic acid, galactonic acid, talonic acid, gluconic acid, and glucoheptonic acid. In various embodiments, the C₂-C₇ aldonic acid comprises a hexonic acid such as gluconic acid. In some embodiments, the C₂-C₇ aldonic acid comprises a pentonic acid. For example, the pentonic acid can be selected from the group consisting of xylonic acid, ribonic acid, arabinonic acid, and mixtures thereof. Lactones of various aldonic acids can also be present and can be formed by intramolecular cyclocondensation of the acids.

Uronic acids, as referred to herein, include monocarboxylic acids of formula (III):

HOOC(HCOH)_{y}CHO (III)

where y can be, for example, as in the invention as claimed an integer from 0 to 10 or, in some embodiments, from 0 to 5. In various embodiments, the uronic acid comprises at least one C₂-C₇ uronic acid. In some embodiments, the uronic acid comprises at least one diuronic aicd, triuronic acid, tetruronic acid, penturonic acid, hexuronic acid, and/or hepturonic acid. Specific C₂-C₇ uronic acids include, for example, glyoxylic acid, glyceruronic acid (tartronaldehydic acid), threuronic acid, erythruronic acid, xylouronic acid, ribouronic acid, arabinuronic acid, lyxonuronic acid, guluronic acid, glucuronic acid, galactouronic acid, mannouronic acid, alluronic acid, altruronic acid, iduronic acid, talonuronic acid, and glucoheptonuronic acid. In various embodiments, the uronic acid comprises a hexuronic acid such as guluronic acid. In some embodiments, the uronic acid comprises a penturonic acid such as xylouronic acid, ribouronic acid, and/or arabinuronic acid. Lactones of various uronic acids can also be present and can be formed by intramolecular cyclocondensation of the acids. Also, the term "uronic acid" and any specific uronic acids mentioned herein and as defined by formula (III) also include cyclic forms (hemiacetal forms) of these compounds.

Aldaric acids, as referred to herein, include dicarboxylic acids of formula (IV):

HOOC(HCOH)_{z}COOH (IV)

where z can be, for example, as in the invention as claimed an integer from 0 to 10 or, in some embodiments, from 0 to 5. In various embodiments, the aldaric acid comprises at least one C₂-C₇ aldaric acid. In some embodiments, the aldaric acid comprises at least one diaric acid, triaric acid, tetraric acid, pentaric acid, hexaric acid, and/or heptaric acid. Specific C₂-C₇ aldaric acids include, for example, oxalic acid, tartronic acid, tartaric acid, xylaric acid, ribaric acid, arabinaric acid, lyxaric acid, allaric acid, altraric acid, glucaric acid, galactaric acid, mannaric acid, gularic acid, idaric acid, talaric acid, and glucoheptaric acid. In some embodiments, the C₂-C₇ aldaric acid comprises a hexaric acid and/or a pentaric acid. In various embodiments, the C₂-C₇ aldaric acid comprises glucaric acid. In further embodiments, the C₂-C₇ aldaric acid comprises a pentaric acid selected from the group consisting of xylaric acid, ribaric acid, arabinaric acid, and mixtures thereof. Lactones of various aldaric acids can also be present and can be formed by intramolecular cyclocondensation of the acids. For example, lactones include glucarolactones such as D-glucaro-1,4-lactone, D-glucaro-6,3-lactone, and D-glucaro-1,4:6,3-dilactone.

Various processes described herein provide for enhanced yields of uronic, aldonic, and aldaric acids, particularly as in the invention as claimed enhanced aldaric acid yields (e.g., enhanced glucaric acid yields). Further, various processes described herein provide for stable product yields over extended operation and/or at high reactor throughputs. These processes can advantageously improve process economics by decreasing the amount of off-path products that may require separation from the product mixture and/or decreasing the amount of on-path products that require further processing.

The preparation of an aldaric acid and/or lactone(s) from an aldonic acid and/or lactone(s) and/or corresponding aldoses is typically a multistep synthesis that proceeds through various intermediates. For example, as shown in the scheme below, the oxidation of glucose to glucaric acid proceeds primarily through two intermediates: gluconic acid and guluronic acid. 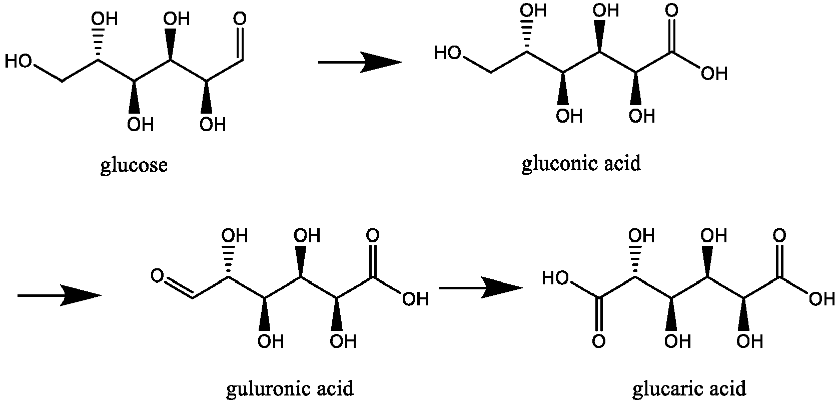 First, the carbonyl of glucose is oxidized to an acid, producing gluconic acid. Next, the terminal hydroxyl at the 6 position of gluconic acid is dehydrogenated to a carbonyl, resulting in guluronic acid. Finally, the carbonyl of guluronic acid is oxidized to an acid, forming glucaric acid.

Previously reported glucaric acid yields (e.g., yields rarely exceeding 70%) may be limited due to a number of factors. Some potential limiting factors include the formation of on-path or off-path products which significantly reduce catalyst efficacy, over-conversion of glucaric acid, and leaching of catalytically active metals. Also, without pH mediation via the continuous introduction of base during the oxidation reaction, the rate of glucaric acid production decreases as the pH decreases. On the other hand, pH mediation results in many cases of lower glucaric acid yields due to the over conversion of glucaric acid to C₂-C₅ sugar acid derivatives. Thus, several inhibiting factors may contribute individually or in combination to limit the total yield to glucaric acid.

In processes for preparing an aldaric acid and/or lactone(s) thereof and/or intermediates thereof by the catalytic oxidation of an aldose, aldonic acid and/or lactone(s) thereof, and/or uronic acid and/or lactones thereof, it has been surprisingly discovered that employing at least two different catalysts provides for enhanced product yields (e.g., aldaric acid yields) even over prolonged operation. It is theorized that by employing at least two different catalysts that some of the inhibiting factors that are expected to limit total yield are reduced or avoided. In particular, it has been discovered that a first catalyst comprising platinum and a second catalyst comprising gold are especially effective for the catalytic oxidation of a C₂-C₇ aldose, C₂-C₇ uronic acid and/or lactone(s) thereof, and C₂-C₇ aldonic acid and/or lactone(s) thereof to the corresponding aldaric acid and/or lactone(s) thereof or intermediate thereof.

### Processes for producing aldaric acids and/or lactone(s) thereof

As noted, embodiments of the present invention include oxidation processes for producing aldaric acids and/or lactone(s) thereof. These processes are as defined in the claims and various processes comprise reacting an aldose of formula (I) to an aldaric acid of formula (IV) and/or lactone(s) thereof in the presence of oxygen, a first catalyst comprising platinum, and a second catalyst comprising gold to form the aldaric acid and/or lactone(s) thereof, wherein the first catalyst and second catalyst are different. These processes can be represented by the following scheme (where w and z are as defined herein):

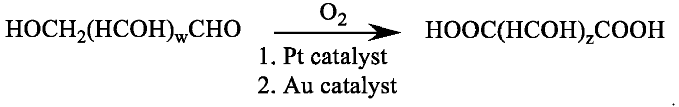

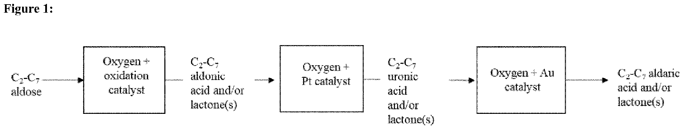

Some embodiments of these processes are directed to processes for preparing C₂-C₇ aldaric acid and/or lactone(s) thereof comprising reacting a C₂-C₇ aldose in the presence of oxygen in a reaction zone comprising an oxidation catalyst, a first catalyst comprising platinum, and a second catalyst comprising gold to form a reaction mixture comprising the C₂-C₇ aldaric acid and/or lactone(s) thereof, wherein the first catalyst and second catalyst are different. For example, when the process is for preparing glucaric acid and/or lactone(s) thereof, the process can comprise reacting glucose in the presence of oxygen in a reaction zone comprising an oxidation catalyst, a first catalyst comprising platinum, and a second catalyst comprising gold to form a reaction mixture comprising the glucaric acid and/or lactone(s) thereof, wherein the first catalyst and second catalyst are different. Figure 1 depicts a diagram showing steps of an oxidation process for preparing a C₂-C₇ aldaric acid and/or lactone(s) thereof from a C₂-C₇ aldose.

Other processes comprise preparing an aldaric acid of formula (IV) and/or lactone(s) thereof comprising reacting an aldonic acid of formula (II) and/or lactone(s) thereof in the presence of oxygen, a first catalyst comprising platinum, and a second catalyst comprising gold to form the aldaric acid and/or lactone(s) thereof, wherein the first catalyst and second catalyst are different. These processes can be represented by the following scheme (where x and z are as defined herein): 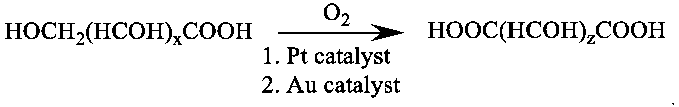 In some embodiments, processes for preparing a C₂-C₇ aldaric acid and/or lactone(s) thereof comprise reacting a C₂-C₇ aldonic acid and/or lactone(s) thereof in the presence of oxygen, a first catalyst comprising platinum, and a second catalyst comprising gold in a reaction zone to form a reaction mixture comprising the C₂-C₇ aldaric acid and/or lactone(s) thereof, wherein the first catalyst and second catalyst are different. For example, when the process is directed to preparing glucaric acid and/or lactone(s) thereof, the process can comprise reacting gluconic acid and/or lactone(s) thereof in the presence of oxygen, a first catalyst comprising platinum, and a second catalyst comprising gold in a reaction zone to form a reaction mixture comprising the glucaric acid and/or lactone(s) thereof, wherein the first catalyst and second catalyst are different.

Embodiments of the present invention also include processes for preparing aldaric acids of formula (IV) and/or lactone(s) thereof comprising reacting a uronic acid of formula (III) and/or lactone(s) thereof in the presence of oxygen, a first catalyst comprising platinum, and a second catalyst comprising gold to form the aldaric acid and/or lactone(s), wherein the first catalyst and second catalyst are different. These processes can be represented by the following scheme (where y and z are as defined herein): 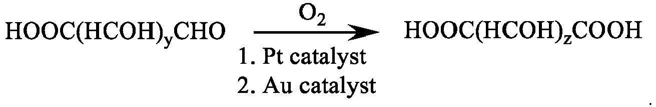 In some embodiments, processes for preparing a C₂-C₇ aldaric acid and/or lactone(s) thereof comprise reacting a C₂-C₇ uronic acid and/or lactone(s) thereof in the presence of oxygen, a first catalyst comprising platinum, and a second catalyst comprising gold in a reaction zone to form a reaction mixture comprising the C₂-C₇ aldaric acid and/or lactone(s), wherein the first catalyst and second catalyst are different. For example, when the process is directed to preparing glucaric acid and/or lactone(s) thereof, the process can comprise reacting guluronic acid and/or lactone(s) thereof in the presence of oxygen, a first catalyst comprising platinum, and a second catalyst comprising gold in a reaction zone to form a reaction mixture comprising the glucaric acid and/or lactone(s), wherein the first catalyst and second catalyst are different.

### Processes for producing uronic acids and/or lactone(s) thereof

Also disclosed are processes for producing uronic acids and/or lactone(s). Various processes comprise preparing a uronic acid of formula (III) and/or lactone(s) thereof comprising reacting an aldose of formula (I) in the presence of oxygen, a first catalyst comprising platinum, and a second catalyst comprising gold to form the uronic acid and/or lactone(s) thereof, wherein the first catalyst and second catalyst are different. These processes can be represented by the following scheme (where w and y are as defined herein):

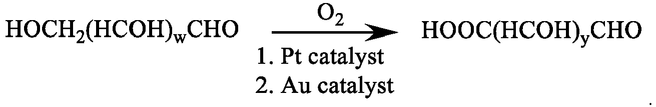

**Such** processes for preparing a C₂-C₇ uronic acid and/or lactone(s) thereof can comprise reacting a C₂-C₇ aldose in the presence of oxygen, a first catalyst comprising platinum, and a second catalyst comprising gold in a reaction zone to form a reaction mixture comprising the C₂-C₇ uronic acid and/or lactone(s), wherein the first catalyst and second catalyst are different. For example, a process for preparing guluronic acid and/or lactone(s) thereof can comprise reacting glucose in the presence of oxygen, a first catalyst comprising platinum, and a second catalyst comprising gold in a reaction zone to form a reaction mixture comprising the guluronic acid and/or lactone(s), wherein the first catalyst and second catalyst are different.

Also disclosed are processes for preparing uronic acids and/or lactone(s) thereof comprising reacting aldonic acids and/or lactone(s) thereof in the presence of oxygen, a first catalyst comprising platinum, and a second catalyst comprising gold to form the uronic acid and/or lactone(s) thereof, wherein the first catalyst and second catalyst are different. These processes can be represented by the following scheme (where x and y are as defined herein):

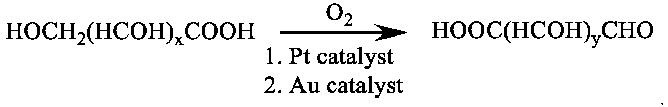

Such processes for preparing a C₂-C₇ uronic acid and/or lactone(s) thereof can comprise reacting a C₂-C₇ aldonic acid and/or lactone(s) thereof in the presence of oxygen, a first catalyst comprising platinum, and a second catalyst comprising gold in a reaction zone to form a reaction mixture comprising the C₂-C₇ uronic acid and/or lactone(s), wherein the first catalyst and second catalyst are different. For example, a process for preparing guluronic acid and/or lactone(s) thereof can comprise reacting gluconic acid and/or lactone(s) thereof in the presence of oxygen, a first catalyst comprising platinum, and a second catalyst comprising gold in a reaction zone to form a reaction mixture comprising the guluronic acid and/or lactone(s), wherein the first catalyst and second catalyst are different.

### Processes for producing aldonic acids and/or lactone(s) thereof

Also disclosed are processes for producing aldonic acids and/or lactone(s). Various processes comprise reacting an aldose of formula (I) to an aldonic acid of formula (II) and/or lactone(s) thereof in the presence of oxygen, a first catalyst comprising platinum, and a second catalyst comprising gold to form the aldonic acid and/or lactone(s) thereof, wherein the first catalyst and second catalyst are different. These processes can be represented by the following scheme (where x and y are as defined herein):

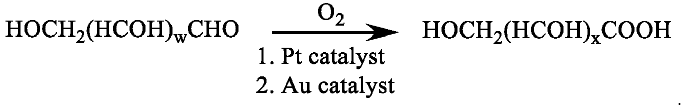

Such processes for preparing a C₂-C₇ aldonic acid and/or lactone(s) thereof can comprise reacting a C₂-C₇ aldose in the presence of oxygen, a first catalyst comprising platinum, and a second catalyst comprising gold in a reaction zone to form a reaction mixture comprising the C₂-C₇ aldonic acid and/or lactone(s), wherein the first catalyst and second catalyst are different. For example, a process for preparing gluconic acid and/or lactone(s) thereof can comprise reacting glucose in the presence of oxygen, a first catalyst comprising platinum, and a second catalyst comprising gold in a reaction zone to form a reaction mixture comprising the gluconic acid and/or lactone(s), wherein the first catalyst and second catalyst are different.

Various processes described herein can further include the step of reacting an aldose of formula (I) in the presence of oxygen and an oxidation catalyst to form an aldonic acid of formula (II) and/or lactone(s) thereof. This additional process step can be represented by the following scheme (where w and x are as defined herein):

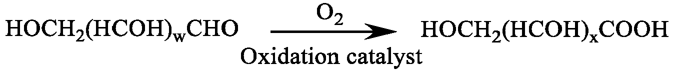

In some embodiments, various processes can further comprise reacting a C₂-C₇ aldose in the presence of oxygen and an oxidation catalyst to form the C₂-C₇ aldonic acid and/or lactone(s) thereof. For example, in processes requiring gluconic acid and/or lactone(s) thereof, these processes can further comprise reacting glucose in the presence of oxygen and an oxidation catalyst to form gluconic acid and/or lactone(s) thereof.

### Additional processes and process features

In various processes described herein, the first catalyst and second catalyst can be mixed. In other words, the reaction zone can comprise a mixture (e.g., physical mixture) of the first catalyst and second catalyst.

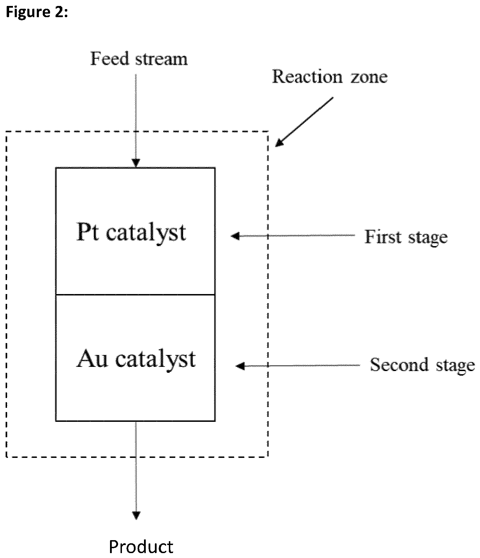

Further, in various processes described herein, the first catalyst and second catalyst can be staged or a combination of mixed and staged. For example, the reaction zone can comprise a first stage comprising the first catalyst and a second stage comprising the second catalyst. See Figure 2, which is a schematic of two-stage reaction zone. Various staged processes for preparing an aldaric acid of formula (IV) and/or lactone(s) thereof by the oxidation an aldonic acid of formula (II) can be represented by the following scheme (where x, y, and z are as defined herein): 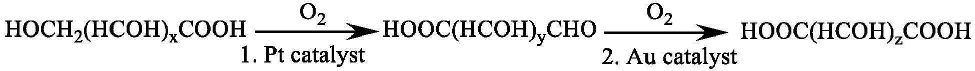 In some embodiments, processes for preparing a C₂-C₇ aldaric acid and/or lactone(s) can comprise reacting a C₂-C₇ aldonic acid and/or lactone(s) thereof in the presence of oxygen and a first catalyst comprising platinum in a first stage of a reaction zone to form a first reaction mixture (e.g., a reaction mixture comprising a C₂-C₇ uronic acid); and contacting the first reaction mixture with oxygen and a second catalyst comprising gold in a second stage of the reaction zone to form a second reaction mixture comprising the C₂-C₇ aldaric acid and/or lactone(s) thereof, wherein the first catalyst and second catalyst are different. When the process is for preparing glucaric acid and/or lactone(s) thereof, the process can comprise reacting gluconic acid and/or lactone(s) thereof in the presence of oxygen and a first catalyst comprising platinum in a first stage of a reaction zone to form a first reaction mixture comprising guluronic acid and/or lactone(s) thereof; and contacting the first reaction mixture comprising guluronic acid and/or lactone(s) thereof with oxygen and a second catalyst comprising gold in a second stage of the reaction zone to form a second reaction mixture comprising the glucaric acid and/or lactone(s) thereof, wherein the first catalyst and second catalyst are different.

In various embodiments comprising a first stage and/or a second stage, the first stage can comprise a mixture of the first catalyst and the second catalyst. In some embodiments, in the first stage, the weight or volume of the first catalyst can be greater than the weight or volume of the second catalyst. For instance, in various embodiments, the first stage comprises a mixture of the first catalyst and the second catalyst and the weight or volumetric ratio of the first catalyst to the second catalyst is about 2:1 or greater, about 3:1 or greater, or about 4: 1 or greater. In some embodiments, the first stage comprises a mixture of the first catalyst and the second catalyst and the weight or volumetric ratio of the first catalyst to the second catalyst can be from about 2:1 to about 10:1, from about 2:1 to about 5:1, from about 3:1 to about 10:1, or from about 3:1 to about 5:1.

In embodiments comprising a first stage and a second stage, the second stage can comprise a mixture of the first catalyst and the second catalyst. In some embodiments, the weight or volume of the second catalyst can be greater than the weight or volume of the first catalyst. For instance, in various embodiments, the second stage comprises a mixture of the first catalyst and the second catalyst and the weight or volumetric ratio of the second catalyst to the first catalyst is about 2:1 or greater, about 3:1 or greater, or about 4:1 or greater. For example, the second stage comprises a mixture of the first catalyst and the second catalyst and the weight or volumetric ratio of the second catalyst to the first catalyst can be from about 2:1 to about 10:1, from about 2:1 to about 5:1, from about 3:1 to about 10:1, or from about 3:1 to about 5:1.

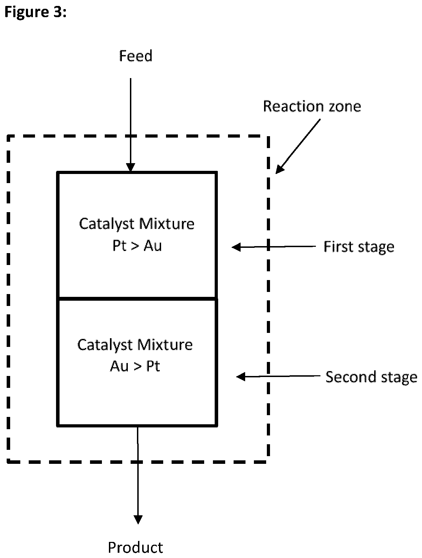

In various embodiments, the first stage and second stage comprise a mixture of the first catalyst and the second catalyst. The stages can comprise different mixtures of the first and second catalyst, including the mixtures note above. Figure 3 depicts a schematic of two-stage reaction zone comprising different catalyst mixtures.

As noted, various processes described herein can further include the step of reacting an aldose in the presence of oxygen and an oxidation catalyst to form the aldonic acid and/or lactone(s) thereof. In these embodiments, the oxidation catalyst, first catalyst, and second catalyst can be staged as well. For example, in some embodiments, the reaction zone can comprise an initial oxidation stage comprising the oxidation catalyst, a first stage comprising the first catalyst, and a second stage comprising the second catalyst.

In various process as described herein, the volumetric ratio of the total amount of first catalyst to second catalyst in the reaction zone is from about 1:10 to about 10:1, from about 1:5 to about 5:1, from about 1:3 to about 3:1, or about 1:1. In further embodiments, the weight or volumetric ratio of the total amount of first catalyst to second catalyst in the reaction zone is from about 1:10 to about 10:1, from about 1:5 to about 5:1, from about 1:3 to about 3:1, or about 1:1.

In various processes described herein, a feed mixture (e.g., a feed solution) can be fed to the reaction zone. In certain embodiments, the feed mixture has an aldose, aldonic acid and/or lactone(s), and/or uronic acid and/or lactone(s) thereof concentration of about 1 wt.% or greater, about 5 wt.% or greater, about 10 wt.% or greater, about 15 wt.% or greater, or about 20 wt.% or greater. In some embodiments, a feed mixture is fed to the reaction zone and the feed mixture has an aldose, aldonic acid and/or lactone(s), and/or uronic acid and/or lactone(s) thereof concentration that is from about 1 wt.% to about 50 wt.%, from about 1 wt.% to about 30 wt.%, from about 1 wt.% to about 25 wt.%, from about 5 wt.% to about 50 wt.%, from about 5 wt.% to about 30 wt.%, from about 5 wt.% to about 25 wt.%, from about 10 wt.% to about 50 wt.%, from about 10 wt.% to about 30 wt.%, from about 10 wt.% to about 25 wt.%, from about 15 wt.% to about 50 wt.%, from about 15 wt.% to about 30 wt.%, from about 15 wt.% to about 25 wt.%, from about 20 wt.% to about 50 wt.%, from about 20 wt.% to about 30 wt.%, or from about 20 wt.% to about 25 wt.%.

In various embodiments, the feed mixture has a C₂-C₇ aldose, C₂-C₇ aldonic acid and/or lactone(s), and/or C₂-C₇ uronic acid and/or lactone(s) thereof concentration of about 1 wt.% or greater, about 5 wt.% or greater, about 10 wt.% or greater, about 15 wt.% or greater, or about 20 wt.% or greater. In some embodiments, a feed mixture is fed to the reaction zone and the feed mixture has an C₂-C₇ aldose, C₂-C₇ aldonic acid and/or lactone(s), and C₂-C₇ uronic acid and/or lactone(s) thereof concentration that is from about 1 wt.% to about 50 wt.%, from about 1 wt.% to about 30 wt.%, from about 1 wt.% to about 25 wt.%, from about 5 wt.% to about 50 wt.%, from about 5 wt.% to about 30 wt.%, from about 5 wt.% to about 25 wt.%, from about 10 wt.% to about 50 wt.%, from about 10 wt.% to about 30 wt.%, from about 10 wt.% to about 25 wt.%, from about 15 wt.% to about 50 wt.%, from about 15 wt.% to about 30 wt.%, from about 15 wt.% to about 25 wt.%, from about 20 wt.% to about 50 wt.%, from about 20 wt.% to about 30 wt.%, or from about 20 wt.% to about 25 wt.%.

In embodiments where the C₂-C₇ aldonic acid and/or lactone(s) comprises gluconic acid and/or lactone(s) thereof, a feed mixture can be fed to the reaction zone and the feed mixture can have a gluconic acid and/or lactone(s) concentration of about 1 wt.% or greater, about 5 wt.% or greater, about 10 wt.% or greater, about 15 wt.% or greater, or about 20 wt.% or greater. In certain embodiments, a feed mixture is fed to the reaction zone and the feed mixture has a gluconic acid and/or lactone(s) concentration that is from about 1 wt.% to about 50 wt.%, from about 1 wt.% to about 30 wt.%, from about 1 wt.% to about 25 wt.%, from about 5 wt.% to about 50 wt.%, from about 5 wt.% to about 30 wt.%, from about 5 wt.% to about 25 wt.%, from about 10 wt.% to about 50 wt.%, from about 10 wt.% to about 30 wt.%, from about 10 wt.% to about 25 wt.%, from about 15 wt.% to about 50 wt.%, from about 15 wt.% to about 30 wt.%, from about 15 wt.% to about 25 wt.%, from about 20 wt.% to about 50 wt.%, from about 20 wt.% to about 30 wt.%, or from about 20 wt.% to about 25 wt.%.

As noted, pH mediation typically results in lower product yields due to the over conversion of aldaric acid to other derivatives. Accordingly, in various embodiments, the processes described herein can be conducted in the absence of added base. In further embodiments, the processes can be conducted wherein the pH of the reaction mixture is not controlled or increased by the addition of base. In some embodiments, the processes can be conducted wherein the reaction mixture is free or essentially free of salt-forming cations.

In various embodiments, the pH of the reaction mixture(s) of the processes described herein, as measured at 20°C, is about 7 or less, about 6.5 or less, about 6 or less, about 5 or less, about 4 or less, about 3 or less, or about 2 or less. For example, the pH of the reaction mixture(s) of the processes described herein, as measured at 20°C, can be from about 1 to about 7, from about 1 to about 6, from about 1 to about 5, from about 1 to about 4, from about 1.5 to about 7, from about 1.5 to about 6, from about 1.5 to about 5, from about 1.5 to about 4, from about 2 to about 7, from about 2 to about 6, from about 2 to about 5, or from about 2 to about 4. In some embodiments, the reaction mixture(s) can reach a minimum pH measured at 20°C of about 4 or less, about 3 or less, or about 2 or less.

In certain embodiments, the reaction zone is heated to a temperature of about 60°C or greater, about 70°C or greater, or about 80°C or greater. For example, the reaction zone can be heated to a temperature of from about 60°C to about 150°C, from about 70°C to about 150°C, from about 80°C to about 150°C, from about 60°C to about 125°C, from about 70°C to about 125°C, from about 80°C to about 125°C, from about 60°C to about 100°C, from about 70°C to about 100°C, or from about 80°C to about 100°C.

In certain embodiments, the oxygen is supplied to the reaction zone as an oxygen-containing gaseous mixture. For example, in some embodiments, the oxygen is supplied to the reaction zone as air, oxygen-enriched air, a mixture comprising oxygen, a mixture comprising at least about 40 or 50 vol.% oxygen, a mixture comprising oxygen and nitrogen (e.g., about 50:50 mixture by volume), or substantially pure oxygen (at least 99 vol.% oxygen). In various embodiments, the oxygen is supplied to the reaction zone as a mixture having an oxygen concentration of about 0.5 vol.% or greater, about 1 vol.% or greater, about 5 vol.% or greater, or about 10 vol.% or greater. For example, in various embodiments, the oxygen is supplied to the reaction zone as a mixture having an oxygen concentration of from about 0.5 vol.% to about 20 vol.%, from about 0.5 vol.% to about 15 vol.%, from about 0.5 vol.% to about 10 vol.%, from about 0.5 vol.% to about 5 vol.%, from about 1 vol.% to about 20 vol.%, from about 1 vol.% to about 15 vol.%, from about 1 vol.% to about 10 vol.%, from about 1 vol.% to about 5 vol.%, from about 5 vol.% to about 20 vol.%, from about 5 vol.% to about 15 vol.%, or from about 5 vol.% to about 10 vol.%. In certain embodiments, the oxygen is supplied to the reaction zone as a mixture having an oxygen concentration of about 10 vol.% or less, about 5 vol.% or less, about 4 vol.% or less, about 3 vol.% or less, about 2 vol.% or less, about 1 vol.% or less, or about 0.5 vol.% or less. For example, from about 10 vol.% to about 0.5 vol.%, from about 5 vol.% to about 0.5 vol.%, from about 4 vol.% to about 0.5 vol.%, from about 3 vol.% to about 0.5 vol.%, from about 2 vol.% to about 0.5 vol.%, or from about 1 vol.% to about 0.5 vol.%.

In various embodiments, the partial pressure of oxygen in the reaction zone is about 115.1 kPa (2 psig) or greater, about 273.7 kPa (25 psig) or greater, about 446.1 kPa (50 psig) or greater, or about 790.8 kPa (100 psig) or greater. For example, the partial pressure of oxygen in the reaction zone can be from about 115.1 kPa (2 psig) to about 13.89 MPa (2000 psig), from about 446.1 kPa (50 psig) to about 13.89 MPa (2000 psig), or from about 790.8 kPa (100 psig) to about 13.89 MPa (2000 psig).

Generally, the processes described herein are catalytic processes, but do not require the application of electric current. Accordingly, in various embodiments, the processes described herein are not electrochemical processes and/or do not comprise applying an electric current to the reaction mixture (e.g., via electrodes).

As noted, processes of the present invention can provide for enhanced product yield. The yield of aldonic acid and/or lactone(s) thereof, uronic acid and/or lactone(s) thereof, and/or aldaric acid and/or lactone(s) thereof may be about 50% or greater, about 55% or greater, about 60% or greater, about 65% or greater, about 70% or greater, or about 75% or greater. For example, the yield of aldonic acid and/or lactone(s) thereof, uronic acid and/or lactone(s) thereof, and/or aldaric acid and/or lactone(s) thereof can be from about 50% to about 85%, from about 50% to about 80%, from about 50% to about 75%, from about 50% to about 70%, from about 50% to about 65%, from about 60% to about 85%, from about 60% to about 80%, from about 60% to about 75%, from about 60% to about 70%, from about 65% to about 85%, from about 65% to about 80%, from about 65% to about 75%, or from about 65% to about 70%.

The yield of C₂-C₇ aldonic acid and/or lactone(s) thereof, C₂-C₇ uronic acid and/or lactone(s) thereof, and/or C₂-C₇ aldaric acid and/or lactone(s) thereof may be about 50% or greater, about 55% or greater, about 60% or greater, about 65% or greater, about 70% or greater, or about 75% or greater. For example, the yield of C₂-C₇ aldonic acid and/or lactone(s) thereof, C₂-C₇ uronic acid and/or lactone(s) thereof, and/or C₂-C₇ aldaric acid and/or lactone(s) thereof can be from about 50% to about 85%, from about 50% to about 80%, from about 50% to about 75%, from about 50% to about 70%, from about 50% to about 65%, from about 60% to about 85%, from about 60% to about 80%, from about 60% to about 75%, from about 60% to about 70%, from about 65% to about 85%, from about 65% to about 80%, from about 65% to about 75%, or from about 65% to about 70%.

In embodiments where the C₂-C₇ aldaric acid and/or lactone(s) comprises glucaric acid and/or lactone(s) thereof, the yield of glucaric acid and/or lactone(s) thereof can be about 50% or greater, about 55% or greater, about 60% or greater, about 65% or greater, about 70% or greater, or about 75% or greater. In various embodiments, the yield of glucaric acid and/or lactone(s) thereof can be from about 50% to about 85%, from about 50% to about 80%, from about 50% to about 75%, from about 50% to about 70%, from about 50% to about 65%, from about 60% to about 85%, from about 60% to about 80%, from about 60% to about 75%, from about 60% to about 70%, from about 65% to about 85%, from about 65% to about 80%, from about 65% to about 75%, or from about 65% to about 70%.

Also, processes of the present invention can provide for stable yield even at high reactor throughput. In certain embodiments, the liquid hourly space velocity (LHSV) of the reaction zone is about 0.2 h⁻¹ or greater, about 0.5 h⁻¹ or greater, about 1 h⁻¹ or greater, about 1.5 h⁻¹ or greater, about 2 h⁻¹ or greater, about 5 h⁻¹ or greater, about 10 h⁻¹ or greater. For example, the LHSV of the reaction zone can be from about 0.2 h⁻¹ to about 50 h⁻¹, from about 0.5 h⁻¹ to about 50 h⁻¹, from about 1 h⁻¹ to about 50 h⁻¹, from about 2 h⁻¹ to about 50 h⁻¹, from about 5 h⁻¹ to about 50 h⁻¹, from about 10 h⁻¹ to about 50 h⁻¹, from about 0.2 h⁻¹ to about 10 h⁻¹, from about 0.5 h⁻¹ to about 10 h⁻¹, from about 1 h⁻¹ to about 10 h⁻¹, from about 2 h⁻¹ to about 10 h⁻¹, or from about 5 h⁻¹ to about 10 h⁻¹. In some embodiments, product yields (e.g., yields of C₂-C₇ aldaric acids, for example glucaric acid, and/or lactone(s) thereof) of at least about 60%, at least about 65%, at least about 70%, or at least about 75% can be achieved at or within the aforementioned LHSV ranges.

The reaction mixture and/or feed mixture can include a solvent. Solvents suitable for the oxidation reaction include, for example, water or aqueous solutions of a carboxylic acid (e.g., acetic acid).

In general, the reaction zone can include one or more batch, semi-batch, or continuous reactor designs using fixed bed reactors, trickle bed reactors, slurry phase reactors, moving bed reactors, or any other design that allows for catalytic reactions, particularly heterogeneous catalytic reactions. Examples of reactors can be seen in Chemical Process Equipment - Selection and Design, Couper et al., Elsevier 1990.

In various processes described herein, the reaction zone comprises one or more trickle bed reactors. It should be understood that reactants, oxygen, any solvent, and the catalysts may be introduced into a suitable reactor separately or in various combinations.

Aldaric, aldonic, and uronic acids and/or lactone(s) thereof produced in accordance with the processes described herein can be converted to various other derivatives, such as salts, esters, ketones, and lactones. Methods to convert carboxylic acids to such derivatives are known in the art, see, for example, Wade, Organic Chemistry 3rd ed., Prentice Hall 1995.

### Catalysts

As noted, one effective catalyst for the oxidation processes described herein comprises platinum (i.e., the first catalyst comprises platinum). This catalyst has been found to be particularly useful for the oxidation of an aldonic acid and/or lactone(s) thereof (e.g., gluconic aid) to a uronic acid and/or lactone(s) thereof (e.g., guluronic acid).

In some embodiments, the first catalyst has a platinum loading of about 10 wt.% or less, about 7.5 wt.% or less, about 5 wt.% or less, about 4 wt.% or less, about 2 wt.% or less, or about 1 wt.% or less. In these and other embodiments, the first catalyst has a platinum loading of about 0.1 wt.% or greater, about 0.25 wt.% or greater, about 0.5 wt.% or greater, about 0.75 wt.% or greater, or about 1 wt.% or greater. For example, in various embodiments, the first catalyst has a platinum loading of from about 0.1 wt.% to about 10 wt.%, from about 0.1 wt.% to about 7.5 wt.%, from about 0.1 wt.% to about 5 wt.%, from about 0.1 wt.% to about 4 wt.%, from about 0.5 wt.% to about 10 wt.%, from about 0.5 wt.% to about 7.5 wt.%, from about 0.5 wt.% to about 5 wt.%, from about 0.5 wt.% to about 4 wt.%, from about 1 wt.% to about 10 wt.%, from about 1 wt.% to about 7.5 wt.%, from about 1 wt.% to about 5 wt.%, from about 1 wt.% to about 4 wt.%, or from about 1 wt.% to about 3 wt.%.

In various embodiments, the first catalyst comprises a catalytically active phase and platinum constitutes a significant portion of the catalytically active phase. For example, in some embodiments, platinum constitutes about 20 wt.% or greater, about 30 wt.% or greater, about 40 wt.% or greater, about 50 wt.% or greater, about 60 wt.% or greater, about 70 wt.% or greater, about 80 wt.% or greater, about 90 wt.% or greater, about 95 wt.% or greater, or about 99 wt.% or greater of the catalytically active phase of the first catalyst. In certain embodiments, platinum constitutes from about 20 wt.% to about 99 wt.%, from about 30 wt.% to about 99 wt.%, from about 40 wt.% to about 99 wt.%, from about 50 wt.% to about 99 wt.%, from about 60 wt.% to about 99 wt.%, from about 70 wt.% to about 99 wt.%, from about 80 wt.% to about 99 wt.%, from about 90 wt.% to about 99 wt.%, from about 95 wt.% to about 99 wt.%, from about 20 wt.% to about 95 wt.%, from about 30 wt.% to about 95 wt.%, from about 40 wt.% to about 95 wt.%, from about 50 wt.% to about 95 wt.%, from about 60 wt.% to about 95 wt.%, from about 70 wt.% to about 95 wt.%, from about 80 wt.% to about 95 wt.%, from about 90 wt.% to about 95 wt.%, from about 20 wt.% to about 90 wt.%, from about 30 wt.% to about 90 wt.%, from about 40 wt.% to about 90 wt.%, from about 50 wt.% to about 90 wt.%, from about 60 wt.% to about 90 wt.%, from about 70 wt.% to about 90 wt.%, or from about 80 wt.% to about 90 wt.% of the catalytically active phase of the first catalyst.

In various embodiments, the first catalyst is a heterogeneous catalyst. In some embodiments, the first catalyst comprises a catalyst support. For example, the support of the first catalyst can comprise a material selected from the group consisting of carbon, alumina, silica, ceria, titania, zirconia, niobia, zeolites, magnesia, clays, nickel, cobalt, copper, iron oxide, silicon carbide, aluminosilicates, montmorillonites, and combinations thereof. In certain embodiments, the support of the first catalyst comprises carbon, titania, zirconia, and combinations thereof. In further embodiments, the support of the first catalyst comprises at least one carbon material selected from the group consisting of graphite, carbon black, activated carbon and combinations thereof. In certain embodiments, the support of the first catalyst comprises carbon black. Various carbon supports and methods of preparing these supports and catalyst compositions are described in U.S. Patent No. 9,682,368 and U.S. Patent Application Publication Nos. 2017/0120223 and 2017/0120219

In various embodiments, the first catalyst can have a BET specific surface area that is at least about 5 m²/g, at least about 100 m²/g, at least about 200 m²/g, at least about 500 m²/g, at least about 1,000 m²/g, at least about 1,500 m²/g, or at least about 2,000 m²/g For example, the first catalyst can have a BET specific surface area that is from about 5 m²/g to about 2,500 m²/g, from about 5 m²/g to about 2,000 m²/g, from about 5 m²/g to about 1,500 m²/g, from about 5 m²/g to about 1,000 m²/g, from about 5 m²/g to about 500 m²/g, from about 5 m²/g to about 200 m²/g, from about 100 m²/g to about 2,500 m²/g, from about 100 m²/g to about 2,000 m²/g, from about 100 m²/g to about 1,500 m²/g, from about 100 m²/g to about 1,000 m²/g, from about 100 m²/g to about 500 m²/g, or from about 100 m²/g to about 200 m²/g.

As noted, a further effective catalyst for the processes described herein comprises gold (i.e., the second catalyst comprises gold). This catalyst has been found to be particularly useful for the oxidation of a uronic acid and/or lactone(s) thereof (e.g., guluronic aid) to an aldaric acid and/or lactone(s) thereof (e.g., glucaric acid).

In some embodiments, the second catalyst has a gold loading of about 10 wt.% or less, about 7.5 wt.% or less, about 5 wt.% or less, about 4 wt.% or less, about 2 wt.% or less, or about 1 wt.% or less. In these and other embodiments, the second catalyst has a gold loading of about 0.1 wt.% or greater, about 0.25 wt.% or greater, about 0.5 wt.% or greater, about 0.75 wt.% or greater, or about 1 wt.% or greater. For example, in various embodiments, the second catalyst has a gold loading of from about 0.1 wt.% to about 10 wt.%, from about 0.1 wt.% to about 7.5 wt.%, from about 0.1 wt.% to about 5 wt.%, from about 0.1 wt.% to about 4 wt.%, from about 0.1 wt.% to about 3 wt.%, from about 0.1 wt.% to about 2 wt.%, from about 0.5 wt.% to about 10 wt.%, from about 0.5 wt.% to about 7.5 wt.%, from about 0.5 wt.% to about 5 wt.%, from about 0.5 wt.% to about 4 wt.%, from about 0.5 wt.% to about 3 wt.%, from about 0.5 wt.% to about 2 wt.%, from about 1 wt.% to about 10 wt.%, from about 1 wt.% to about 7.5 wt.%, from about 1 wt.% to about 5 wt.%, from about 1 wt.% to about 4 wt.%, from about 1 wt.% to about 3 wt.%, or from about 1 wt.% to about 2 wt.%.

In various embodiments, the second catalyst comprises a catalytically active phase and gold constitutes a significant portion of the catalytically active phase. For example, in some embodiments, gold constitutes about 20 wt.% or greater, about 30 wt.% or greater, about 40 wt.% or greater, about 50 wt.% or greater, about 60 wt.% or greater, about 70 wt.% or greater, about 80 wt.% or greater, about 90 wt.% or greater, about 95 wt.% or greater, or about 99 wt.% or greater of the catalytically active phase of the second catalyst. In certain embodiments, gold constitutes from about 20 wt.% to about 99 wt.%, from about 30 wt.% to about 99 wt.%, from about 40 wt.% to about 99 wt.%, from about 50 wt.% to about 99 wt.%, from about 60 wt.% to about 99 wt.%, from about 70 wt.% to about 99 wt.%, from about 80 wt.% to about 99 wt.%, from about 90 wt.% to about 99 wt.%, from about 95 wt.% to about 99 wt.%, from about 20 wt.% to about 95 wt.%, from about 30 wt.% to about 95 wt.%, from about 40 wt.% to about 95 wt.%, from about 50 wt.% to about 95 wt.%, from about 60 wt.% to about 95 wt.%, from about 70 wt.% to about 95 wt.%, from about 80 wt.% to about 95 wt.%, from about 90 wt.% to about 95 wt.%, from about 20 wt.% to about 90 wt.%, from about 30 wt.% to about 90 wt.%, from about 40 wt.% to about 90 wt.%, from about 50 wt.% to about 90 wt.%, from about 60 wt.% to about 90 wt.%, from about 70 wt.% to about 90 wt.%, or from about 80 wt.% to about 90 wt.% of the catalytically active phase of the second catalyst.

In various embodiments, the second catalyst is a heterogeneous catalyst. In some embodiments, the second catalyst can comprise a catalyst support. For example, the support of the second catalyst can comprise a material selected from the group consisting of carbon, alumina, silica, ceria, titania, zirconia, niobia, zeolites, magnesia, clays, nickel, cobalt, copper, iron oxide, silicon carbide, aluminosilicates, montmorillonites, and combinations thereof. In some embodiments, the support of the second catalyst comprises a material comprising carbon, alumina, silica, titania, zirconia, and combinations thereof. In further embodiments, the support of the second catalyst comprises at least one carbon material selected from the group consisting of graphite, carbon black, activated carbon and combinations thereof (e.g., a carbon black support as noted herein). In certain embodiments, the support of the second catalyst comprises zirconia, doped zirconia, doped zirconia-metal composite, doped zirconia-metal oxide composite, titania, doped titania, doped titania-metal composite, doped titania-metal oxide composite, and mixtures thereof. In various embodiments, the support of the second catalyst comprises titania. In certain embodiments, the support of the second catalyst is not the same as the support of the first catalyst.

In various embodiments, the second catalyst can have a BET specific surface area that is at least about 5 m²/g, at least about 100 m²/g, at least about 200 m²/g, at least about 500 m²/g, at least about 1,000 m²/g, at least about 1,500 m²/g, or at least about 2,000 m²/g. For example, the second catalyst can have a BET specific surface area that is from about 5 m²/g to about 2,500 m²/g, from about 5 m²/g to about 2,000 m²/g, from about 5 m²/g to about 1,500 m²/g, from about 5 m²/g to about 1,000 m²/g, from about 5 m²/g to about 500 m²/g, from about 5 m²/g to about 200 m²/g, from about 100 m²/g to about 2,500 m²/g, from about 100 m²/g to about 2,000 m²/g, from about 100 m²/g to about 1,500 m²/g, from about 100 m²/g to about 1,000 m²/g, from about 100 m²/g to about 500 m²/g, or from about 100 m²/g to about 200 m²/g.

As noted, various processes of the present invention can further comprise reacting an aldose (e.g., glucose) in the presence of oxygen and an oxidation catalyst. In these embodiments, the oxidation catalyst can include, for example, the first catalyst or second catalyst as described herein or any combination thereof. In some embodiments, the oxidation catalyst comprises the second catalyst as described herein.

When a catalyst support is used, the metals (e.g., platinum and gold) may be deposited on the catalyst supports using procedures known in the art including, but not limited to incipient wetness, ion-exchange, deposition-precipitation, and vacuum impregnation.

### EXAMPLES

The following non-limiting examples are provided to further illustrate the present invention.

### Example 1: Glucose to gluconic acid using a 1 wt.% Au/TiO₂ catalyst (Reference Example)

A 1 wt.% Au/TiO₂ catalyst crushed and sieved to achieve a 40/80 mesh size, weighing approximately 27 g, and measuring approximately 30 cm³ was loaded into a fixed packed bed reactor. A 20 wt.% glucose solution in water was fed to the reactor at a liquid hourly space velocity (LHSV) of 2.0 h⁻¹ and a gas flow of 75% N₂ and 25% air was fed co-currently at a rate of 1000 SCCM. The pressure of the system was maintained at 5.27 MPa (750 psig). The jacket temperature of the reactor was set to 77°C. The space time yield (STY) (g[GA]/g [PGM]*h) was 43.

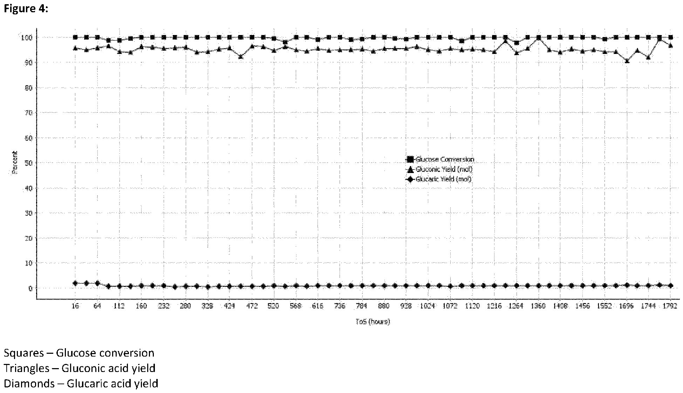

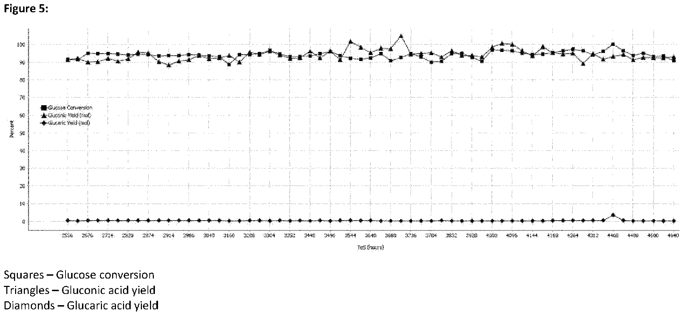

Glucose conversion and gluconic acid production were stable over the course of the observation. The peak internal temperature at the radial center of the catalyst bed was 88-89°C. The average gluconic acid yield was about 94 mol.%, with an average glucaric acid yield of less than about 0.5 mol.%. A plot of the glucose conversion, gluconic acid yield (mol %), and glucaric acid yield (mol %) is shown in Figures 4 and 5.

This example demonstrates that a 1 wt.% Au/TiO₂ catalyst, while suitable for the formation of gluconic acid, is unable to convert said gluconic acid to glucaric acid at any appreciable amount.

### Example 2: Gluconic acid oxidation using Pt catalysts (Reference Example)

A 4 wt.% Pt/C catalyst sized to 40/80 mesh (30 cm³) was loaded in to a fixed packed bed reactor. A 21.5 wt.% gluconic acid solution in water was fed to the reactor at a liquid hourly space velocity (LHSV) of 1.0 h⁻¹ and a gas flow of air was fed co-currently at a rate of 1000 SCCM. The pressure of the system was maintained at 5.27 MPa (750 psig). The jacket temperature of the reactor was varied between 60-90°C.

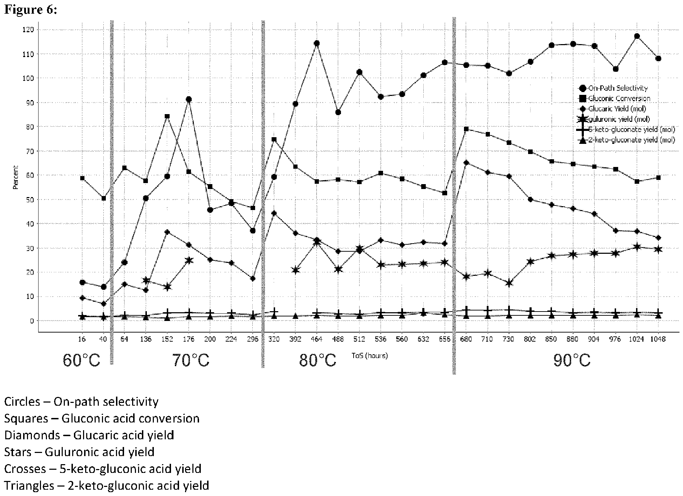

Figure 6 reports the various conversion, selectivity, and yields achieved during oxidation at these varying temperatures. A maximum glucaric acid yield of 65% was observed at a temperature of 90°C, but decreased to approximately 35% during the course of reaction at this temperature. A significant yield of guluronic acid (approximately 10-30 mol.%) was observed during the course of this reaction.

Table 1 presents detailed information regarding the conversion and yield using a 4 wt.% Pt/C catalyst at various temperature and air flow rates. The pressure was maintained at 5.27 MPa (750 psig) and the LHSV of the feed stream was maintained at 1 for the data reported below.

**Table 1**

| Time on stream (h) | Reactor jacket T (°C) | Air flow (SCCM) | N₂ flow (SCCM) | Gluconic acid feed (wt.%) | Gluconic acid conv. (%) | Glucaric acid yield (mol.%) | Guluronic acid yield (mol.%) | 2-keto gluconic yield (mol.%) | 5-keto gluconic acid yield (mol.%) |
|---|---|---|---|---|---|---|---|---|---|
| 16 | 60 | 996 | 1 | 21.5 | 58.7 | 9.3 | - | 1.7 | 1.9 |
| 40 | 60 | 995 | 3 | 21.5 | 50.4 | 7.0 | - | 1.5 | 1.6 |
| 152 | 70 | 992 | 2 | 21.5 | 84.5 | 36.5 | 13.8 | 1.0 | 3.1 |
| 176 | 70 | 997 | 2 | 21.5 | 61.4 | 31.1 | 24.9 | 1.7 | 3.3 |
| 200 | 70 | 994 | 5 | 21.5 | 55.1 | 25.2 | - | 1.7 | 3.0 |
| 224 | 70 | 997 | 5 | 21.5 | 49.3 | 23.9 | - | 1.9 | 3.0 |
| 296 | 70 | 995 | 4 | 21.5 | 46.5 | 17.3 | - | 1.7 | 2.4 |
| 320 | 80 | 992 | 3 | 21.5 | 74.7 | 44.3 | - | 1.8 | 3.7 |
| 392 | 80 | 993 | 0 | 21.5 | 63.5 | 36.1 | 20.7 | 1.9 | - |
| 464 | 80 | 992 | 0 | 21.5 | 57.5 | 33.5 | 32.2 | 2.1 | 3.1 |
| 488 | 80 | 999 | 2 | 21.5 | 58.1 | 28.7 | 21.2 | 1.9 | 2.8 |
| 512 | 80 | 996 | 4 | 21.5 | 57.0 | 28.6 | 29.9 | 1.8 | 2.6 |
| 536 | 80 | 996 | 3 | 21.5 | 61.0 | 33.2 | 23.1 | 2.1 | 3.3 |
| 560 | 80 | 990 | 4 | 21.5 | 58.4 | 31.2 | 23.3 | 2.1 | 3.2 |
| 632 | 80 | 999 | 3 | 19.7 | 55.2 | 32.3 | 23.6 | 3.3 | 3.4 |
| 656 | 80 | 994 | 3 | 19.7 | 52.5 | 31.7 | 24.1 | 2.4 | 3.4 |
| 680 | 90 | 994 | 4 | 19.7 | 79.1 | 65.2 | 18.1 | 1.8 | 4.3 |
| 710 | 90 | 993 | 4 | 19.7 | 76.8 | 61.2 | 19.6 | 2.0 | 4.2 |
| 730 | 90 | 991 | 4 | 19.7 | 73.4 | 59.4 | 15.4 | 2.1 | 4.5 |
| 802 | 90 | 1000 | 4 | 19.7 | 69.6 | 50.0 | 24.3 | 2.1 | 3.9 |
| 850 | 90 | 997 | 3 | 19.7 | 65.7 | 47.7 | 26.8 | 2.2 | 3.9 |
| 880 | 90 | 993 | 4 | 19.7 | 64.6 | 46.3 | 27.4 | 2.2 | 3.2 |
| 904 | 90 | 994 | 4 | 19.7 | 63.5 | 44.1 | 27.7 | 2.2 | 3.5 |
| 976 | 90 | 1005 | 4 | 21.3 | 62.6 | 37.2 | 27.7 | 2.1 | 3.2 |
| 1024 | 90 | 984 | 3 | 21.3 | 57.3 | 36.8 | 30.4 | 2.3 | 3.4 |
| 1048 | 90 | 996 | 3 | 21.3 | 58.9 | 34.2 | 29.5 | 2.2 | 3.1 |

A 4 wt.% Pt/TiO₂ catalyst was also tested to determine the suitability for conversion of gluconic acid to glucaric acid. As with the Pt/C catalyst, the overall glucaric acid yield degraded over time. The glucaric acid yield range is presented in the table below, which details the maximum yield achieved and the final yield observed. A comparison of the results of these catalysts at 80°C is presented below in Table 2.

**Table 2**

| Catalyst | 4% Pt/C | 4% Pt/TiO₂ |
|---|---|---|
| T (°C) | 80 | 80 |
| Pₜₒₜₐₗ (psig)* | 750 | 750 |
| P_{O2} (psig) * | 150 | 150 |
| LHSV (h⁻¹) | 1 | 0.5 |
| Gluconic acid (wt.%) | 21.5 | 10 |
| Glucaric yield (mol.%) | 44-29 | 47-19 |
| STY (g[GA]/g [PGM]*h) | 9 | 0.8 |

| | | |
|---|---|---|
| *** 1 psig = 108.22 kPa** | | |

This example demonstrates the poor stability and significant degradation of glucaric acid yield observed when gluconic acid is oxidized solely in the presence of a 4 wt.% Pt/C or 4 wt.% Pt/TiO₂ catalyst.

### Example 3: Glucose to glucaric acid using a Pt-Au/TiO₂ catalyst

A platinum-gold catalyst was prepared by loading platinum on a Au/TiO₂ catalyst such that the final catalyst comprised 1 wt.% Pt and 1 wt.% Au. A fixed packed bed reactor was loaded with the catalyst. A 10 wt.% glucose in water feed was introduced into the reactor at an initial LHSV of 1.0 h⁻¹ and a gas flow of 25% air and 75% N₂ was introduced co-currently at a rate of 1000 SCCM. The initial temperature of the reactor was 60°C and the initial partial pressure of oxygen was maintained at 359.9 kPa (37.5 psig). The STY (g[Glucaric acid]/g[PGM]*h) was 6.

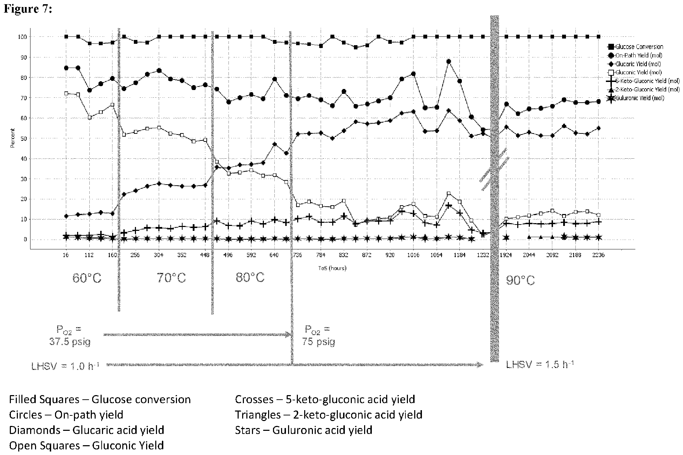

As noted in Figure 7, over the course of 2236 hours, the temperature was gradually increased from 60°C to 90°C in increments of 10°C. At approximately 640 hours, the partial pressure of oxygen was increased from 359.9 kPa (37.5 psig) to 618.43 kPa (75 psig) by changing the gas composition to 50% air and 50% N₂ while keeping the total gas flow rate at 1000 SCCM. At approximately 1232 hours, the 10 wt.% glucose feed was increased from a LHSV of 1.0 h⁻¹ to a LHSV of 1.5 h⁻¹. Stable operation was observed at 90°C, a total pressure of 5.27 MPa (750 psig), and a LHSV of 1.5 h⁻¹.

Results of the experiment are shown below in Tables 3 and 4. The total pressure was maintained at 5.27 MPa (750 psig) throughout.

**Table 3**

| Time on stream (h) | Reactor jacket T (°C) | LHSV | Air flow (SCCM) | N₂ flow (SCCM) | Glucose feed (wt.%) | Glucose conversion (%) |
|---|---|---|---|---|---|---|
| 16-60 | 62 | 1.0 | 249 | 757 | 10 | 98 |
| 184-448 | 70 | 1.0 | 249 | 756 | 10 | 99 |
| 472-664 | 80 | 1.0 | 249 | 754 | 10 | 99 |
| 736-1208 | 80 | 1.0 | 499 | 510 | 10 | 98 |
| 1924-2574 | 90 | 1.5 | 630 | 416 | 10 | 100 |
| 2646-2838 | 90 | 1.5 | 998 | 154 | 20 | 100 |

**Table 4**

| Time on stream (h) | Gluconic acid yield (mol.%) | Glucaric acid yield (mol.%) | Guluronic acid yield (mol.%) | 2-keto gluconic yield (mol.%) | 5-keto gluconic acid yield (mol.%) |
|---|---|---|---|---|---|
| 16-60 | 63 | 15 | 1 | 0 | 2 |
| 184-448 | 52 | 26 | 0 | 0 | 5 |
| 472-664 | 33 | 39 | 0 | 0 | 8 |
| 736-1208 | 14 | 56 | 1 | 2 | 10 |
| 1924-2574 | 11 | 56 | 1 | 1 | 8 |
| 2646-2838 | 26 | 47 | 3 | 1 | 10 |

### Example 4: Gluconic acid oxidation using a physical mixture of 4 wt.% Pt/C and 1 wt.% Au/TiO₂ catalysts

The catalyst of Example 1 (1 wt.% Au/TiO₂) was physically mixed with the catalyst of Example 2 (4 wt.% Pt/C) in a volumetric ratio of 1:1, forming a 30 cm³ catalyst bed. The mass ratio of the catalysts (Au/TiO₂:Pt/C) was approximately 3:2.

A 20 wt.% gluconic acid in water feed was passed over the catalyst mixture at a LHSV of 0.5 h⁻¹ with a co-current gas flow rate of 100% air at 1000 SCCM. The pressure was maintained at 5.27 MPa (750 psig) and the LHSV of the feed stream was maintained at 0.5 h⁻¹.

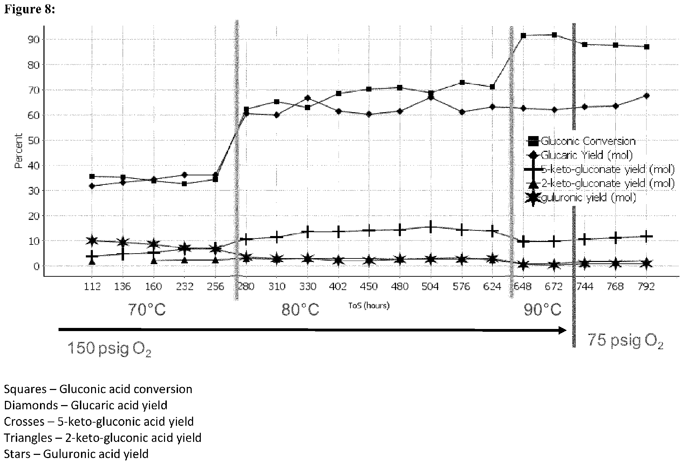

Figure 8 reports the gluconic acid conversion and various yields achieved during the reaction at temperatures between 70°C and 90°C. After 672 hours while at 90°C, the gas flow was changed from 100% air to 50% air with 50% N₂. The total gas flow was maintained at 1000 SCCM.

Stable production of glucaric acid (i.e. a yield greater than about 60 mol.%) was observed at 80°C and 90°C. This is compared to the decreasing 44-29 mol.% yield of glucaric acid when using Pt/C alone at 80°C (Example 2) and 35-45 mol.% yield when using a catalyst comprising Pt loaded on Au/TiO₂ at 80°C (Example 3). The processes using Pt/C alone or Pt loaded on Au/TiO₂ either failed to achieve a desirable yield or achieved a maximum yield that was not sustained (e.g., the Pt/C yield severely degrading over time at 90°C).

Table 5 presents detailed information regarding the conversion and yield using a mixture of Pt/C and Au/TiO₂ catalysts at various temperature and air flow rates.

**Table 5**

| Time on stream (h) | Reactor jacket T (°C) | Air flow (SCCM) | N₂ flow (SCCM) | Gluconic acid feed (wt.%) | Gluconic acid conv. (%) | Glucaric acid yield (mol.%) | Guluronic acid yield (mol.%) | 2-keto gluconic yield (mol.%) | 5-keto gluconic acid yield (mol.%) |
|---|---|---|---|---|---|---|---|---|---|
| 64 | 60 | 998 | 0 | 19.1 | 11.0 | 16.9 | 20.1 | 1.7 | 2.5 |
| 88 | 60 | 998 | 0 | 19.1 | 16.1 | 13.0 | 11.7 | 1.4 | 2.2 |
| 112 | 70 | 998 | 0 | 19.1 | 35.7 | 31.8 | 10.1 | 1.7 | 3.9 |
| 136 | 70 | 998 | 0 | 19.1 | 35.3 | 33.3 | 9.3 | - | 4.8 |
| 160 | 70 | 998 | 0 | 19.1 | 33.9 | 34.3 | 8.7 | 2.2 | 5.3 |
| 232 | 70 | 998 | 0 | 19.1 | 32.7 | 36.3 | 7.1 | 2.4 | 6.8 |
| 256 | 70 | 998 | 0 | 19.1 | 34.4 | 36.1 | 6.7 | 2.4 | 6.9 |
| 280 | 80 | 998 | 0 | 19.1 | 62.4 | 60.7 | 3.5 | 3.0 | 10.6 |
| 310 | 80 | 998 | 0 | 19.1 | 65.2 | 60.1 | 2.8 | 2.6 | 11.4 |
| 330 | 80 | 998 | 0 | 19.1 | 63.0 | 66.7 | 2.9 | 3.0 | 13.7 |
| 402 | 80 | 998 | 0 | 19.1 | 68.7 | 61.5 | 2.2 | 3.0 | 13.7 |
| 450 | 80 | 998 | 0 | 19.1 | 70.5 | 60.5 | 2.0 | 2.9 | 14.1 |
| 480 | 80 | 998 | 0 | 19.1 | 70.9 | 61.5 | 2.7 | 2.8 | 14.3 |
| 504 | 80 | 998 | 0 | 19.1 | 69.0 | 67.1 | 2.8 | 2.8 | 15.5 |
| 576 | 80 | 998 | 0 | 20.2 | 72.9 | 61.2 | 2.5 | 3.1 | 14.3 |
| 624 | 80 | 998 | 0 | 20.2 | 71.3 | 63.4 | 3.1 | 2.3 | 14.0 |
| 648 | 90 | 998 | 0 | 20.2 | 91.6 | 62.8 | 0.4 | 1.0 | 9.7 |
| 672 | 90 | 998 | 0 | 20.2 | 91.8 | 62.1 | 0.3 | 0.9 | 9.8 |
| 744 | 90 | 500 | 497 | 20.2 | 87.9 | 63.3 | 0.8 | 1.9 | 10.7 |
| 768 | 90 | 500 | 497 | 20.2 | 87.6 | 63.6 | 0.9 | 1.8 | 11.3 |
| 792 | 90 | 499 | 497 | 20.2 | 87.1 | 67.6 | 0.9 | 1.9 | 11.8 |

### Example 5: Gluconic acid to glucaric acid using a staged reactor bed comprising a physical mixture of catalysts

Gluconic acid was converted to glucaric acid using a mixture of Pt/C and Au/TiO₂ catalysts. A staged reactor bed was prepared comprising two physical mixtures of catalysts. The upper stage comprised a 4:1 (vol) ratio of 4 wt.% Pt/C and 1 wt.% Au/TiO₂. The lower stage comprised a 1:4 (vol) ratio of 4 wt.% Pt/C and 1 wt.% Au/TiO₂. A 20 wt.% gluconic acid in water feed stream was introduced as a LHSV of 1.0 h⁻¹. At approximately 624 hours, the reactor temperature was increased from 90°C to 95°C.

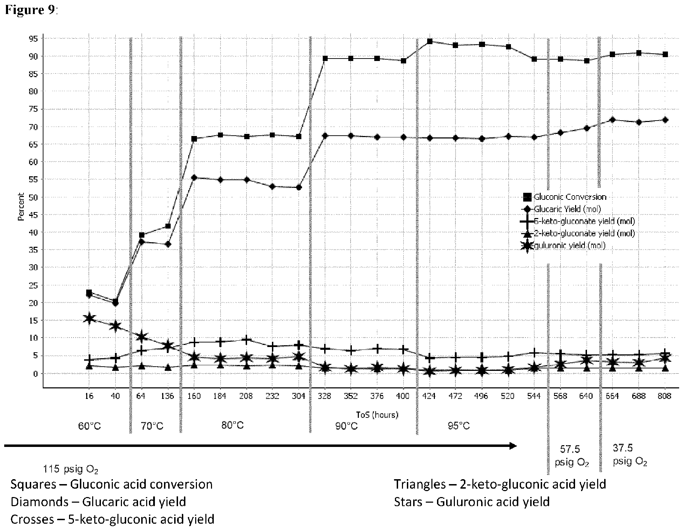

As shown in Figure 9, glucaric acid yield of 65-72 mol.% was achieved, with a noticeable increase in yield when the temperature was increased. The conversion of gluconic acid averaged in excess of 90% for the duration of the 808 hour reaction.

Table 6 presents detailed information regarding the conversion and yield using a mixture of Pt/C and Au/TiO₂ catalysts at various temperature and air flow rates.

**Table 6**

| Time on stream (h) | Reactor jacket T (°C) | Pressure (psig) * | Air flow (SCCM) | N₂ flow (SCCM) | Gluconic acid conv. (%) | Glucaric acid yield (mol.%) | Guluronic acid yield (mol.%) | 2-keto gluconic yield (mol.%) | 5-keto gluconic acid yield (mol.%) |
|---|---|---|---|---|---|---|---|---|---|
| 16 | 60 | 1150 | 491.5 | 498.6 | 23.0 | 22.2 | 15.6 | 2.0 | 3.8 |
| 40 | 60 | 1150 | 467.4 | 498.5 | 20.5 | 19.8 | 13.4 | 1.7 | 4.3 |
| 64 | 71 | 1150 | 464.5 | 503.0 | 39.1 | 37.2 | 10.2 | 2.1 | 6.3 |
| 136 | 71 | 1150 | 476.3 | 494.3 | 41.8 | 36.6 | 7.8 | 1.8 | 7.1 |
| 160 | 80 | 1150 | 440.7 | 498.8 | 66.5 | 55.5 | 4.5 | 2.3 | 8.7 |
| 184 | 80 | 1150 | 470.2 | 500.6 | 67.5 | 54.8 | 4.1 | 2.3 | 8.8 |
| 208 | 80 | 1150 | 440.3 | 504.7 | 67.2 | 54.9 | 4.3 | 2.1 | 9.4 |
| 232 | 80 | 1150 | 462.0 | 495.8 | 67.7 | 53.0 | 4.1 | 2.2 | 7.5 |
| 304 | 80 | 1150 | 498.4 | 500.7 | 67.1 | 52.7 | 4.6 | 2.2 | 7.9 |
| 328 | 90 | 1150 | 498.6 | 502.2 | 89.3 | 67.4 | 1.7 | 1.4 | 6.8 |
| 352 | 90 | 1150 | 497.7 | 499.8 | 89.3 | 67.4 | 1.2 | 1.4 | 6.4 |
| 376 | 90 | 1150 | 499.1 | 510.2 | 89.4 | 67.0 | 1.7 | 1.3 | 6.9 |
| 400 | 90 | 1150 | 498.9 | 505.0 | 88.7 | 67.0 | 1.3 | 1.4 | 6.7 |
| 424 | 95 | 1150 | 498.5 | 497.5 | 94.2 | 66.7 | 0.5 | 0.8 | 4.3 |
| 472 | 95 | 1150 | 498.6 | 499.5 | 92.9 | 66.7 | 0.8 | 1.0 | 4.4 |
| 496 | 95 | 1150 | 498.4 | 500.5 | 93.2 | 66.5 | 0.9 | 0.9 | 4.4 |
| 520 | 95 | 1150 | 498.5 | 499.6 | 92.6 | 67.3 | 1.1 | 0.9 | 4.7 |
| 544 | 95 | 1150 | 248.3 | 757.9 | 89.2 | 67.0 | 1.7 | 1.5 | 5.7 |
| 568 | 95 | 1150 | 249.1 | 742.1 | 89.2 | 68.3 | 2.6 | 1.5 | 5.4 |
| 640 | 95 | 1150 | 236.7 | 748.8 | 88.8 | 69.5 | 3.7 | 1.5 | 5.0 |
| 664 | 95 | 750 | 249.1 | 758.1 | 90.3 | 72.0 | 3.1 | 1.5 | 5.2 |
| 688 | 95 | 750 | 248.9 | 744.6 | 90.9 | 71.2 | 3.1 | 1.5 | 5.2 |
| 808 | 95 | 750 | 249.0 | 756.0 | 90.3 | 71.8 | 4.2 | 1.5 | 5.5 |

| | | | | | | | | | |
|---|---|---|---|---|---|---|---|---|---|
| *1 psig = 108.22 kPa | | | | | | | | | |

### Example 6: Gluconic acid oxidation using a physical mixture of 4 wt.% Pt/C and 1 wt.% Au/TiO₂ catalysts

The experiment of Example 4 was modified such that the pressure was maintained at 8.72 MPa (1250 psig) and the jacket temperature of the reactor was maintained at 85°C. A stream of 21.2 wt.% gluconic acid in water was introduced at a LHSV of 0.5 h⁻¹ and a gas stream (50 % N₂ and 50% air) was fed at a flow rate of 1000 SCCM.

At approximately 1224 hours, the catalyst was washed by stopping the liquid gluconic acid feed and starting a water feed, while maintaining the gas flow and temperature as set forth above. This was done at a LHSV of 1.0 h⁻¹ for 4 hours. After this time, the flow of gluconic acid feed was resumed.

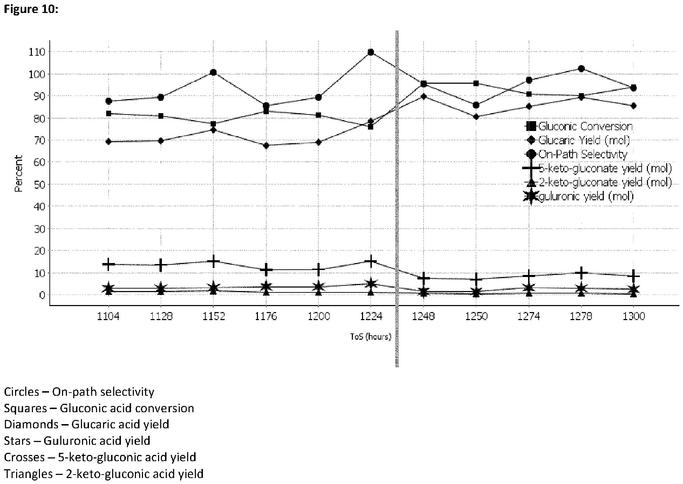

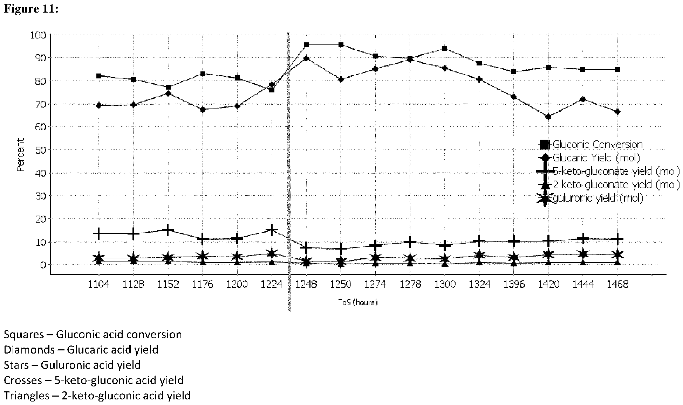

Figure 10 and Table 7 reports the glucaric acid yield before and after the water washing step. An increase in the glucaric acid yield was observed immediately after the washing step, and continued for approximately 75 hours before slightly decreasing. Figure 11 reports the results for 1104-1468 hours.

**Table 7**

| Time on stream (h) | Air flow (SCCM) | N₂ flow (SCCM) | Gluconic acid conversion (%) | Glucaric acid yield (mol.%) | Guluronic acid yield (mol.%) | 2-keto gluconic yield (mol.%) | 5-keto gluconic acid yield (mol.%) |
|---|---|---|---|---|---|---|---|
| 1104-1224 | 748.8 | 300.2 | 80.1 | 71.4 | 3.5 | 1.3 | 13.3 |
| 1248-1300 | 499.1 | 498.1 | 93.2 | 86.1 | 2.2 | 0.6 | 8.2 |

### Example 7: Arabinose to arabinonic acid using a 1 wt.% Au/TiO₂ catalyst (Reference Example)

A 1 wt.% Au/TiO₂ catalyst crushed and sieved to achieve a 40/80 mesh size, weighing approximately 27 g, and measuring approximately 30 cm³ was loaded into a fixed packed bed reactor. A 20.6 wt.% arabinose solution in water was fed to the reactor at a liquid hourly space velocity (LHSV) of 1.0 h⁻¹ and a gas flow of 50% N₂ and 50% air was fed co-currently at a rate of 1000 SCCM. The pressure of the system was maintained at 5.27 MPa (750 psig). The jacket temperature of the reactor was set to 75°C. Six data points were taken from this experiment between a total time on stream of 2716 to 2828 hours.

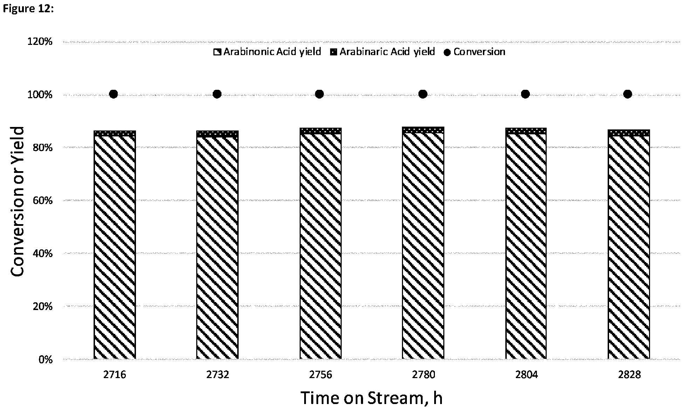

Arabinose conversion and arabinonic acid production were stable over the course of the observation. Conversion of approximately 100% was observed for each data point. The arabinonic acid yield was between about 84 and 86 mol.%, with an average arabinaric acid yield of less than about 2.2 mol.%. Figure 12 reports the arabinose conversion, arabinonic acid yield (mol %), and arabinaric acid yield (mol %) for each data point. A summary of the results are set forth below in Table 8.

**Table 8**

| Time on Stream (h) | Arabinonic Acid Yield (mol. %) | Arabinaric Acid Yield (mol. %) | Conversion (%) |
|---|---|---|---|
| 2716 | 85 | 1.7 | 100.0 |
| 2732 | 84 | 2.1 | 100.0 |
| 2756 | 85 | 2.1 | 100.0 |
| 2780 | 86 | 2.2 | 100.0 |
| 2804 | 85 | 2.2 | 100.0 |
| 2828 | 85 | 2.1 | 100.0 |

### Example 8: Arabinonic acid to arabinaric acid using a staged reactor bed comprising a physical mixture of catalysts

Arabinonic acid was converted to arabinaric acid using a mixture of Pt/C and Au/TiO₂ catalysts. A staged reactor bed was prepared comprising two physical mixtures of catalysts. The upper stage comprised a 4:1 (vol) ratio of 4 wt.% Pt/C and 1 wt.% Au/TiO₂. The lower stage comprised a 1:4 (vol) ratio of 4 wt.% Pt/C and 1 wt.% Au/TiO₂. A 19.4 wt.% arabinonic acid in water feed stream was introduced at a LHSV of 1.0 h⁻¹. A co-current gas flow rate of 25% air and 75% N₂ was introduced at 1000 SCCM. The initial reactor pressure was 8.38 MPa (1200 psig) and the initial reactor temperatures was 100°C. Six data points were taken from this experiment between a total time on stream of 856 hours to 938 hours.

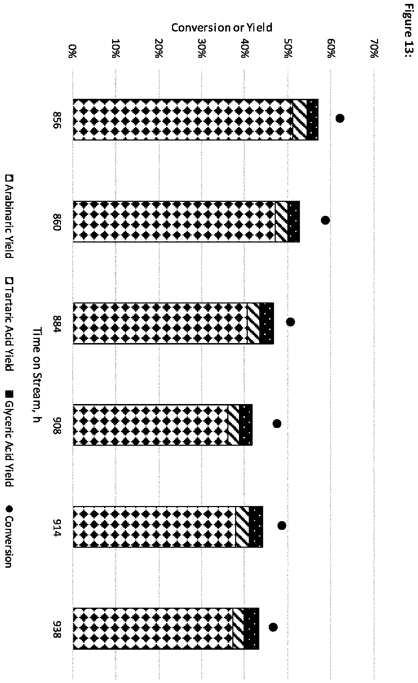

Arabinaric acid yield of 36-51 mol.% was achieved and the conversion of arabinonic acid was approximately 46-62%. Figure 13 reports the arabinonic acid conversion, arabinaric acid yield (mol %), tartaric acid yield (mol %), and glyceric acid yield (mol %) for each measurement. A summary of the results are set forth below in Table 9.

**Table 9**

| Time on Stream (h) | Arabinonic acid conversion | Arabinaric acid yield (mol. %) | Tartaric acid yield (mol. %) | Glyceric acid yield (mol. %) |
|---|---|---|---|---|
| 856 | 62 | 51 | 3 | 2 |
| 860 | 59 | 47 | 3 | 3 |
| 884 | 50 | 41 | 3 | 3 |
| 908 | 47 | 36 | 3 | 3 |
| 914 | 48 | 38 | 3 | 3 |
| 938 | 46 | 37 | 3 | 3 |

### Example 9: Glyceraldehyde to glyceric acid using an Au/TiO₂ catalyst (Reference Example)

A 1 wt.% Au/TiO₂ catalyst crushed and sieved to achieve a 40/80 mesh size weighing approximately 27 g and measuring approximately 30 cm³ was loaded into a fixed packed bed reactor. A 1.22 wt.% glyceraldehyde solution in water was fed to the reactor at a liquid hourly space velocity (LHSV) of 1.0 h⁻¹ and a gas flow of 50% N₂ and 50% air was fed co-currently at a rate of 1000 SCCM. The pressure of the system was maintained at 5.27 MPa (750 psig).
The jacket temperature of the reactor was set to 75°C. Four data points were taken from this experiment between a total time on stream of 3000 to 3038 hours.

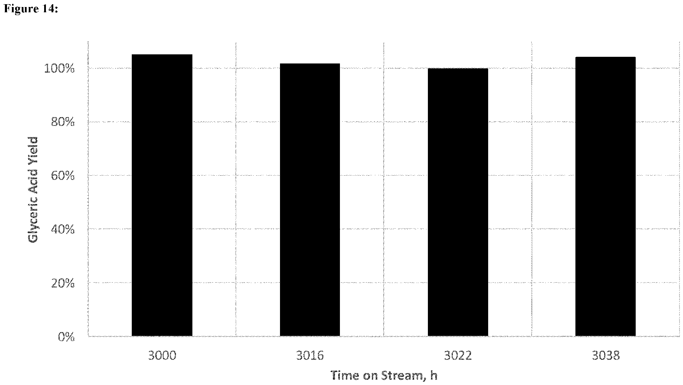

Glyceraldehyde conversion and glyceric acid production were stable over the course of the observation. The glyceric acid yield was between about 100 and 105 mol.%, with an average tartronic acid yield of 0 mol.%. Figure 14 reports the glyceric acid yield (mol %). A summary of the results are set forth below in Table 10.

**Table 10**

| Time on Stream (h) | Glyceric acid yield (mol. %) | Tartronic Acid Yield (mol. %) |
|---|---|---|
| 3000 | 105 | 0 |
| 3016 | 102 | 0 |
| 3022 | 100 | 0 |
| 3038 | 104 | 0 |

### Example 10: Glyceric acid to tartronic acid using a staged reactor bed comprising a physical mixture of catalysts

Glyceric acid was converted to tartronic acid using a mixture of Pt/C and Au/TiO₂ catalysts. A staged reactor bed was prepared comprising two physical mixtures of catalysts. The upper stage comprised a 4:1 (vol) ratio of 4 wt.% Pt/C and 1 wt.% Au/TiO₂. The lower stage comprised a 1:4 (vol) ratio of 4 wt.% Pt/C and 1 wt.% Au/TiO₂. A 10.0 wt.% glyceric acid in water feed stream was introduced at a LHSV as noted below in Table 11. A co-current gas flow rate of air and N₂ was introduced at approximately 1000 SCCM in the ratio noted below. The initial reactor pressure was 5.27 MPa (750 psig) and was increased to 8.72 MPa (1250 psig) during the experiment. Similarly, the initial reactor temperatures was 95°C and increased to 106°C during the course of the experiment.

**Table 11**

| Time on Stream (h) | Jacket Temp. (°C) | Nitrogen Flow (SCCM) | Nominal Pressure (psig)* | Air Flow (SCCM) | LHSV | Glyceric Acid Conv. (%) | Tartronic Acid Yield (mol.%) | Glycolic Acid Yield (mol.%) | Oxalic Acid Yield (mol.%) | Formic Acid Yield (mol.%) |
|---|---|---|---|---|---|---|---|---|---|---|
| 175 | 95 | 500 | 750 | 500 | 1 | 43 | 0.5 | 8.3 | 5.0 | 1.0 |
| 177 | 95 | 499 | 750 | 500 | 2 | 18 | 0.4 | 8.2 | 2.0 | 0.4 |
| 179 | 106 | 899 | 1250 | 101 | 2 | 19 | 0.4 | 8.5 | 2.3 | 0.2 |
| 180 | 106 | 499 | 1250 | 499 | 2 | 41 | 0.8 | 9.0 | 3.3 | 1.1 |

| | | | | | | | | | | |
|---|---|---|---|---|---|---|---|---|---|---|
| * 750 psig = 5.27 MPa 1250 psig = 8.72 MPa | | | | | | | | | | |

### Example 11: Glycolaldehyde to glycolic acid using various catalysts (Reference Example)

Experiments were conducted to test the efficacy of various catalysts for oxidation of glycoaldehyde to glycolic acid. One experiment was conducted without the use of a catalyst. The other experiments utilized an 1 wt.% Au/TiOz catalyst, 4 wt.% Pt/C catalyst, or physical mixture of 4 wt.% Pt/C and 1 wt.% Au/TiO₂ catalysts.

The 1 wt.% Au/TiO₂ catalyst and 4 wt.% Pt/C catalyst were crushed and sieved to achieve a 40/80 mesh size. The total catalyst weight used for each experiment is reflected below in Table 12. The experiments comprising a physical mixture of 4 wt.% Pt/C and 1 wt.% Au/TiO₂ catalysts utilized a weight ratio of the catalysts of 1:1.

For each experiment, the amount of catalyst noted below in Table 12 was loaded in to a batch reactor. The batch reactor was loaded with a 6.1 wt.% glycolaldehyde solution in water and pressurized to 12.51 MPa (1800 psig) using 100% air. The jacket temperature of the reactor was set to 85°C. The total volume of the liquid in each reactor experiment was 2.3 mL.

**Table 12**

| Experiment Number | Catalyst | Total Catalyst Mass (mg) | Glycolaldehyde Conversion (%) | Glycolic Acid Yield (mol. %) | Glycolic Acid (mol. %) |
|---|---|---|---|---|---|
| 1 | No catalyst | 0 | 0 | 0 | 0.0 |
| 2 | | 0 | 5 | 0 | 0.0 |
| 3 | 1 wt.% Au/TiO₂ | 100 | 69 | 39 | 3.0 |
| 4 | | 100 | 67 | 39 | 3.0 |
| 5 | | 200 | 68 | 39 | 3.0 |
| 6 | | 200 | 68 | 39 | 3.0 |
| 7 | 4 wt.% Pt/C | 100 | 48 | 57 | 4.4 |
| 8 | | 100 | 51 | 57 | 4.4 |
| 9 | | 200 | 44 | 58 | 4.5 |
| 10 | | 200 | 45 | 60 | 4.6 |
| 11 | Physical Mixture: 4 wt.% Pt/C + 1 wt.% Au/TiO₂ | 100 | 42 | 66 | 5.1 |
| 12 | | 100 | 41 | 67 | 5.2 |
| 13 | | 200 | 41 | 63 | 4.9 |
| 14 | | 200 | 46 | 64 | 4.9 |

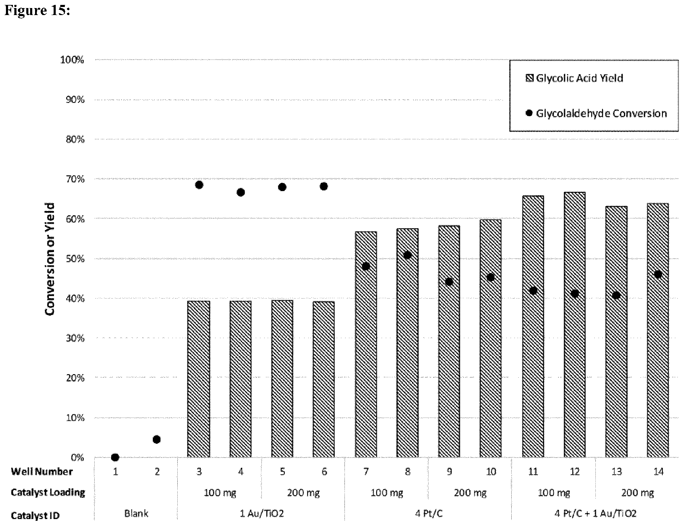

The results of these experiments are reported in Figure 15. As expected, the results without the use of a catalyst were poor. The 1 wt.% Au/TiO₂ catalyst exhibited high conversion and a glycolic acid yield (mol.%) of approximately 40%. The 4 wt.% Pt/C catalyst exhibited a lower conversion than the 1 wt.% Au/TiO₂ catalyst, but a higher glycolic acid yield. The physical mixture of catalysts exhibited a higher glycolic acid yield than either of the catalysts alone.

### Example 12: Glycolic acid to oxalic acid using a staged reactor bed comprising a physical mixture of catalysts

Glycolic acid was converted to oxalic acid using a mixture of Pt/C and Au/TiO₂ catalysts. A staged reactor bed was prepared comprising two physical mixtures of catalysts. The upper stage comprised a 4:1 (vol) ratio of 4 wt.% Pt/C and 1 wt.% Au/TiO₂. The lower stage comprised a 1:4 (vol) ratio of 4 wt.% Pt/C and 1 wt.% Au/TiO₂. A 10.3 wt.% glycolic acid in water feed stream was introduced at a LHSV as set forth below. A co-current gas flow was introduced at approximately 1000 SCCM. Table 13, below, indicates the gas flow composition at various times on stream. The initial reactor pressure was 8.72 MPa (1250 psig) and the initial reactor j acket temperatures was 75°C and varied as reported below in Table 13. Results are reported below for a time on stream of 1222-1280 hours.

**Table 13**

| Time on Stream (h) | Jacket Temperature (°C) | Nitrogen Flow (SCCM) | Air Flow (SCCM) | LHSV | Glycolic Acid Conversion (%) | Oxalic Acid Yield (mol.%) |
|---|---|---|---|---|---|---|
| 1222 | 75 | 749 | 250 | 1.0 | 51 | 0.04 |
| 1224 | 75 | 749 | 250 | 1.0 | 57 | 0.00 |
| 1226 | 58 | 749 | 250 | 1.0 | 27 | 0.00 |
| 1242 | 58 | 749 | 250 | 1.0 | 14 | 0.00 |
| 1246 | 75 | 899 | 100 | 1.0 | 39 | 0.22 |
| 1248 | 75 | 899 | 100 | 2.0 | 22 | 0.23 |
| 1272 | 76 | 899 | 100 | 2.0 | 23 | 0.15 |
| 1276 | 80 | 899 | 100 | 2.0 | 28 | 1.69 |
| 1278 | 85 | 898 | 100 | 2.0 | 27 | 4.25 |
| 1280 | 90 | 898 | 100 | 2.0 | 27 | 3.69 |

Low oxalic acid yields are believed to be due to over-conversion of the intermediate glyoxal and/or oxalic acid to COz. This is supported by the fact that no glyoxal was observed in the reaction product and elevated COz levels were measured. It appears an elevated LHSV (i.e. from 1248-1280 hours on stream) resulted in some oxalic acid exiting the reactor before having the opportunity to be over-converted.

When introducing elements of the present invention or the preferred embodiment(s) thereof, the articles "a", "an", "the" and "said" are intended to mean that there are one or more of the elements. The terms "comprising", "including" and "having" are intended to be inclusive and mean that there may be additional elements other than the listed elements.

In view of the above, it will be seen that the several objects of the invention are achieved and other advantageous results attained.

As employed herein, the term "comprising" is to be understood to also cover the alternative in which the product/method/use in respect of which the term "comprising" is used may also "consist exclusively of" the subsequently-described elements.

As employed herein, the term "comprising" is to be understood to also cover the alternative in which the product/method/use in respect of which the term "comprising" is used may also "consist essentially of" the subsequently-described elements.

Unless stated otherwise, all synthetic processes and parameter measurements are to be understood to have been conducted at room/ambient temperature, i.e. at 21±1 °C.

## Claims

1. A process for preparing an aldaric acid and/or lactone(s) thereof, the process comprising:
reacting
(i) an aldose of formula (I) and/or lactone(s) thereof:
HOCH₂(HCOH)_{w}CHO (I)
wherein w is an integer from 0 to 10; or
(ii) an aldonic acid of formula (II) and/or lactone(s) thereof:
HOCH₂(HCOH)ₓCOOH (II)
where x is an integer from 0 to 10; or
(iii) a uronic acid of formula (III) and/or lactone(s) thereof:
HOOC(HCOH)_{y}CHO (III)
wherein y is an integer from 0 to 10;
in the presence of oxygen, a first catalyst comprising platinum, and a second catalyst comprising gold in a reaction zone to form a reaction mixture comprising the aldaric acid of formula (IV) and/or lactone(s) thereof, wherein the first catalyst and second catalyst are different and
wherein the aldaric acid and/or lactone(s) thereof is of formula (IV):
HOOC(HCOH)_{z}COOH (IV)
wherein z is an integer from 0 to 10; and
wherein in alternative (i) the reaction zone further comprises an oxidation catalyst.

2. The process according to claim 1, wherein
in alternative (i) w and/or z is an integer from 0 to 5;
in alternative (ii) x and/or z is an integer from 0 to 5; and
in alternative (iii) y and/or z is an integer from 0 to 5.

3. The process according to any of the preceding claims, wherein the aldaric acid of formula (IV) comprises at least one C₂-C₇ aldaric acid.

4. The process of any of the preceding claims, wherein the aldaric acid of formula (IV) comprises triaric acid, tetraric acid, pentaric acid, hexaric acid, heptaric acid, and/or a mixture thereof.

5. The process of any of the preceding claims, wherein the aldaric acid of formula (IV) comprises oxalic acid, tartronic acid, tartaric acid, xylaric acid, ribaric acid, arabinaric acid, glucaric acid, galactaric acid, mannaric acid, and/or a mixture thereof.

6. The process of any one of the preceding claims, wherein the aldaric acid of formula (IV) comprises glucaric acid, xylaric acid, ribaric acid, arabinaric acid, and/or a mixture thereof.

7. The process according to any of the preceding claims, wherein
in alternative (i), the aldose of formula (I) is glucose and the aldaric acid of formula (IV) is glucaric acid;
in alternative (ii), the aldonic acid of formula (II) is gluconic acid and the aldaric acid of formula (IV) is glucaric acid; and
in alternative (iii), the uronic acid of formula (III) is guluronic acid and the aldaric acid of formula (IV) is glucaric acid.

8. The process of any one of the preceding claims, wherein the reaction zone comprises a first stage and the first stage comprises a mixture of the first catalyst and the second catalyst and the weight or volume of the first catalyst is greater than the weight or volume of the second catalyst.

9. The process of any one of the preceding claims, wherein the reaction zone comprises a second stage and the second stage comprises a mixture of the first catalyst and the second catalyst and the weight or volume of the second catalyst is greater than the weight or volume of the first catalyst.

10. The process of any one of the preceding claims, wherein the first catalyst has a platinum loading of from about 0.1 wt.% to about 10 wt.%, from about 0.1 wt.% to about 7.5 wt.%, from about 0.1 wt.% to about 5 wt.%, from about 0.1 wt.% to about 4 wt.%, from about 0.5 wt.% to about 10 wt.%, from about 0.5 wt.% to about 7.5 wt.%, from about 0.5 wt.% to about 5 wt.%, from about 0.5 wt.% to about 4 wt.%, from about 1 wt.% to about 10 wt.%, from about 1 wt.% to about 7.5 wt.%, from about 1 wt.% to about 5 wt.%, from about 1 wt.% to about 4 wt.%, or from about 1 wt.% to about 3 wt.%.

11. The process of any one of the preceding claims, wherein the second catalyst has a gold loading of from about 0.1 wt.% to about 10 wt.%, from about 0.1 wt.% to about 7.5 wt.%, from about 0.1 wt.% to about 5 wt.%, from about 0.1 wt.% to about 4 wt.%, from about 0.1 wt.% to about 3 wt.%, from about 0.1 wt.% to about 2 wt.%, from about 0.5 wt.% to about 10 wt.%, from about 0.5 wt.% to about 7.5 wt.%, from about 0.5 wt.% to about 5 wt.%, from about 0.5 wt.% to about 4 wt.%, from about 0.5 wt.% to about 3 wt.%, from about 0.5 wt.% to about 2 wt.%, from about 1 wt.% to about 10 wt.%, from about 1 wt.% to about 7.5 wt.%, from about 1 wt.% to about 5 wt.%, from about 1 wt.% to about 4 wt.%, from about 1 wt.% to about 3 wt.%, or from about 1 wt.% to about 2 wt.%.

12. The process of any one of the preceding claims, wherein the LHSV of the reaction zone is from about 0.2 h⁻¹ to about 50 h⁻¹, from about 0.5 h⁻¹ to about 50 h⁻¹, from about 1 h⁻¹ to about 50 h⁻¹, from about 2 h⁻¹ to about 50 h⁻¹, from about 5 h⁻¹ to about 50 h⁻¹, from about 10 h⁻¹ to about 50 h⁻¹, from about 0.2 h⁻¹ to about 10 h⁻¹, from about 0.5 h⁻¹ to about 10 h⁻¹, from about 1 h⁻¹ to about 10 h⁻¹, from about 2 h⁻¹ to about 10 h⁻¹, or from about 5 h⁻¹ to about 10 h⁻¹.

13. The process of any one of the preceding claims, wherein the reaction zone is heated to a temperature of from about 60°C to about 150°C, from about 70°C to about 150°C, from about 80°C to about 150°C, from about 60°C to about 125°C, from about 70°C to about 125°C, from about 80°C to about 125°C, from about 60°C to about 100°C, from about 70°C to about 100°C, or from about 80°C to about 100°C.

14. The process of any one of the preceding claims, wherein the partial pressure of oxygen is in the range of from about 2 psig to about 2000 psig (from about 115.1 kPa to about 13.89 MPa), from about 50 psig to about 2000 psig (from about 446.1 kPa to about 13.89 MPa), or from about 100 psig to about 2000 psig (from about 790.8 kPa to about 13.89 MPa).

15. The process of any one of the preceding claims, wherein the oxygen is supplied to the reaction zone as air, oxygen-enriched air, a mixture comprising at least about 40 or 50 vol.% oxygen, a mixture comprising oxygen and nitrogen, or substantially pure oxygen.

## Patentansprüche

1. Verfahren zur Herstellung einer Aldarsäure und/oder Lactone(n) derselben, wobei das Verfahren umfasst:
das Umsetzen
(i) einer Aldose der Formel (I) und/oder Lactone(n) derselben:
HOCH₂(HCOH)_{w}CHO (I)
wobei w eine ganze Zahl von 0 bis 10 ist; oder
(ii) eine Aldonsäure der Formel (II) und/oder Lactone(n) derselben:
HOCH₂(HCOH)ₓCOOH (II)
wobei x eine ganze Zahl von 0 bis 10 ist; oder
(iii) einer Uronsäure der Formel (III) und/oder Lactone(n) derselben:
HOOC(HCOH)_{y}CHO (III)
wobei y eine ganze Zahl von 0 bis 10 ist;
in Gegenwart von Sauerstoff, eines ersten Katalysators, der Platin umfasst, und eines zweiten Katalysators, der Gold umfasst, in einer Reaktionszone unter Bildung eines Reaktionsgemischs; das die Aldarsäure der Formel (IV) und/oder das oder die Lactone davon umfasst, wobei der erste Katalysator und der zweite Katalysator voneinander verschieden sind und wobei die Aldarsäure und/oder das oder die Lactone davon der Formel (IV) entsprechen:
HOOC(HCOH)_{z}COOH (IV)
wobei z eine ganze Zahl von 0 bis 10 ist; und
wobei in Alternative (i) die Reaktionszone weiterhin einen Oxidationskatalysator umfasst.

2. Verfahren gemäß Anspruch 1, wobei
in Alternative (i) w und/oder z eine ganze Zahl von 0 bis 5 ist;
in Alternative (ii) x und/oder z eine ganze Zahl von 0 bis 5 ist; und
in Alternative (iii) y und/oder z eine ganze Zahl von 0 bis 5 ist.

3. Verfahren gemäß einem der vorstehenden Ansprüche, wobei die Aldarsäure der Formel (IV) wenigstens eine C₂-C₇-Aldarsäure umfasst.

4. Verfahren gemäß einem der vorstehenden Ansprüche, wobei die Aldarsäure der Formel (IV) Triarsäure, Tetrarsäure, Pentarsäure, Hexarsäure, Heptarsäure und/oder ein Gemisch davon umfasst.

5. Verfahren gemäß einem der vorstehenden Ansprüche, wobei die Aldarsäure der Formel (IV) Oxalsäure, Tartronsäure, Weinsäure, Xylarsäure, Ribarsäure, Arabinarsäure, Glucarsäure, Galactarsäure, Mannarsäure und/oder ein Gemisch davon umfasst.

6. Verfahren gemäß einem der vorstehenden Ansprüche, wobei die Aldarsäure der Formel (IV) Glucarsäure, Xylarsäure, Ribarsäure, Arabinarsäure und/oder ein Gemisch davon umfasst.

7. Verfahren gemäß einem der vorstehenden Ansprüche, wobei
in Alternative (i) die Aldose der Formel (I) Glucose ist und die Aldarsäure der Formel (IV) Glucarsäure ist;
in Alternative (ii) die Aldonsäure der Formel (II) Gluconsäure ist und die Aldarsäure der Formel (IV) Glucarsäure ist; und
in Alternative (iii) die Uronsäure der Formel (III) Guluronsäure ist und die Aldarsäure der Formel (IV) Glucarsäure ist.

8. Verfahren gemäß einem der vorstehenden Ansprüche, wobei die Reaktionszone eine erste Stufe umfasst und die erste Stufe ein Gemisch aus dem ersten Katalysator und dem zweiten Katalysator umfasst und das Gewicht oder das Volumen des ersten Katalysators größer ist als das Gewicht oder das Volumen des zweiten Katalysators.

9. Verfahren gemäß einem der vorstehenden Ansprüche, wobei die Reaktionszone eine zweite Stufe umfasst und die zweite Stufe ein Gemisch aus dem ersten Katalysator und dem zweiten Katalysator umfasst und das Gewicht oder das Volumen des zweiten Katalysators größer ist als das Gewicht oder das Volumen des ersten Katalysators.

10. Verfahren gemäß einem der vorstehenden Ansprüche, wobei der erste Katalysator einen Platinanteil von etwa 0,1 Gew.-% bis etwa 10 Gew.-%, von etwa 0,1 Gew.-% bis etwa 7,5 Gew.-%, von etwa 0,1 Gew.-% bis etwa 5 Gew.-%, von etwa 0,1 Gew.-% bis etwa 4 Gew.-%, von etwa 0,5 Gew.-% bis etwa 10 Gew.-%, von etwa 0,5 Gew.-% bis etwa 7,5 Gew.-%, von etwa 0,5 Gew.-% bis etwa 5 Gew.-%, von etwa 0,5 Gew.-% bis etwa 4 Gew.-%, von etwa 1 Gew.-% bis etwa 10 Gew.-%, von etwa 1 Gew.-% bis etwa 7,5 Gew.-%, von etwa 1 Gew.-% bis etwa 5 Gew.-%, von etwa 1 Gew.-% bis etwa 4 Gew.-% oder von etwa 1 Gew.-% bis etwa 3 Gew.-% aufweist.

11. Verfahren gemäß einem der vorstehenden Ansprüche, wobei der zweite Katalysator einen Goldanteil von etwa 0,1 Gew.-% bis etwa 10 Gew.-%, von etwa 0,1 Gew.-% bis etwa 7,5 Gew.-%, von etwa 0,1 Gew.-% bis etwa 5 Gew.-%, von etwa 0,1 Gew.-% bis etwa 4 Gew.-%, von etwa 0,1 Gew.-% bis etwa 3 Gew.-%, von etwa 0,1 Gew.-% bis etwa 2 Gew.-%, von etwa 0,5 Gew.-% bis etwa 10 Gew.-%, von etwa 0,5 Gew.-% bis etwa 7,5 Gew.-%, von etwa 0,5 Gew.-% bis etwa 5 Gew.-%, von etwa 0,5 Gew.-% bis etwa 4 Gew.-%, von etwa 0,5 Gew.-% bis etwa 3 Gew.-%, von etwa 0,5 Gew.-% bis etwa 2 Gew.-%, von etwa 1 Gew.-% bis etwa 10 Gew.-%, von etwa 1 Gew.-% bis etwa 7,5 Gew.-%, von etwa 1 Gew.-% bis etwa 5 Gew.-%, von etwa 1 Gew.-% bis etwa 4 Gew.-%, von etwa 1 Gew.-% bis etwa 3 Gew.-% oder von etwa 1 Gew.-% bis etwa 2 Gew.-% aufweist.

12. Verfahren gemäß einem der vorstehenden Ansprüche, wobei das LHSV der Reaktionszone etwa 0,2 h⁻¹ bis etwa 50 h⁻¹, etwa 0,5 h⁻¹ bis etwa 50 h⁻¹, etwa 1 h⁻¹ bis etwa 50 h⁻¹, etwa 2 h⁻¹ bis etwa 50 h⁻¹, etwa 5 h⁻¹ bis etwa 50 h⁻¹, etwa 10 h⁻¹ bis etwa 50 h⁻¹, etwa 0,2 h⁻¹ bis etwa 10 h⁻¹, etwa 0,5 h⁻¹ bis etwa 10 h⁻¹, etwa 1 h⁻¹ bis etwa 10 h⁻¹, etwa 2 h⁻¹ bis etwa 10 h⁻¹ oder etwa 5 h⁻¹ bis etwa 10 h⁻¹ beträgt.

13. Verfahren gemäß einem der vorstehenden Ansprüche, wobei die Reaktionszone auf eine Temperatur von etwa 60°C bis etwa 150°C, etwa 70°C bis etwa 150°C, etwa 80°C bis etwa 150°C, etwa 60°C bis etwa 125°C, etwa 70°C bis etwa 125°C, etwa 80°C bis etwa 125°C, etwa 60°C bis etwa 100°C, etwa 70°C bis etwa 100°C oder etwa 80°C bis etwa 100°C erhitzt ist.

14. Verfahren gemäß einem der vorstehenden Ansprüche, wobei der Partialdruck von Sauerstoff im Bereich von etwa 2 psi Manometerdruck bis etwa 2000 psi Manometerdruck (etwa 115,1 kPa bis etwa 13,89 MPa), von etwa 50 psi Manometerdruck bis etwa 2000 psi Manometerdruck (etwa 446,1 kPa bis etwa 13,89 MPa) oder von etwa 100 psi Manometerdruck bis etwa 2000 psi Manometerdruck (etwa 790,8 kPa bis etwa 13,89 MPa) liegt.

15. Verfahren gemäß einem der vorstehenden Ansprüche, wobei der Sauerstoff der Reaktionszone in Form von Luft, mit Sauerstoff angereicherter Luft, eines Gemischs, das wenigstens etwa 40 oder 50 Vol.-% Sauerstoff umfasst, einem Gemisch, das Sauerstoff und Stickstoff umfasst, oder im Wesentlichen reinem Sauerstoff zugeführt wird.

## Revendications

1. Procédé de préparation d'un acide aldarique et/ou de lactone(s) de celui-ci, le procédé comprenant :
la mise en réaction
(i) d'un aldose de formule (I) et/ou de lactone(s) de celui-ci :
HOCH₂(HCOH)_{w}CHO (I)
où w est un nombre entier allant de 0 à 10 ; ou
(ii) d'un acide aldonique de formule (II) et/ou de lactone(s) de celui-ci :
HOCH₂(HCOH)ₓCOOH (II)
où x est un nombre entier allant de 0 à 10 ; ou
(iii) d'un acide uronique de formule (III) et/ou de lactone(s) de celui-ci :
HOOC(HCOH)_{y}CHO (III)
où y est un nombre entier allant de 0 à 10 ;
en présence d'oxygène, d'un premier catalyseur comprenant du platine, et d'un second catalyseur comprenant de l'or dans une zone de réaction pour former un mélange réactionnel comprenant l'acide aldarique de formule (IV) et/ou des lactone(s) de celui-ci, dans lequel le premier catalyseur et le second catalyseur sont différents et dans lequel l'acide aldarique et/ou des lactones de celui-ci répond/répondent à la formule (IV) :
HOOC(HCOH)_{z}COOH (IV)
où z est un nombre entier allant de 0 à 10 ; et
dans lequel dans la variante (i), la zone de réaction comprend en outre un catalyseur d'oxydation.

2. Procédé selon la revendication 1, dans lequel
dans la variante (i), w et/ou z est/sont un nombre entier allant de 0 à 5 ;
dans la variante (ii), x et/ou z est/sont un nombre entier allant de 0 à 5 ; et
dans la variante (iii) y et/ou z est/sont un nombre entier allant de 0 à 5.

3. Procédé selon l'une des revendications précédentes, dans lequel l'acide aldarique de formule (IV) comprend au moins un acide aldarique en C₂ à C₇.

4. Procédé de l'une des revendications précédentes, dans lequel l'acide aldarique de formule (IV) comprend l'acide triarique, l'acide tétrarique, l'acide pentarique, l'acide hexarique, l'acide heptarique et/ou un mélange de ceux-ci.

5. Procédé de l'une des revendications précédentes, dans lequel l'acide aldarique de formule (IV) comprend l'acide oxalique, l'acide tartronique, l'acide tartrique, l'acide xylarique, l'acide ribarique, l'acide arabinarique, l'acide glucarique, l'acide galactarique, l'acide mannarique, et/ou un mélange de ceux-ci.

6. Procédé de l'une quelconque des revendications précédentes, dans lequel l'acide aldarique de formule (IV) comprend l'acide glucarique, l'acide xylarique, l'acide ribarique, l'acide arabinarique et/ou un mélange de ceux-ci.

7. Procédé selon l'une des revendications précédentes, dans lequel dans la variante (i), l'aldose de formule (I) est le glucose et l'acide aldarique de formule (IV) est l'acide glucarique ;
dans la variante (ii), l'acide aldonique de formule (II) est l'acide gluconique et l'acide aldarique de formule (IV) est l'acide glucarique ; et
dans la variante (iii), l'acide uronique de formule (III) est l'acide guluronique et l'acide aldarique de formule (IV) est l'acide glucarique.

8. Procédé de l'une quelconque des revendications précédentes, dans lequel la zone de réaction comprend un premier étage et le premier étage comprend un mélange du premier catalyseur et du second catalyseur et le poids ou le volume du premier catalyseur est supérieur au poids ou au volume du second catalyseur.

9. Procédé de l'une quelconque des revendications précédentes, dans lequel la zone de réaction comprend un second étage et le second étage comprend un mélange du premier catalyseur et du second catalyseur et le poids ou le volume du second catalyseur est supérieur au poids ou au volume du premier catalyseur.

10. Procédé de l'une quelconque des revendications précédentes, dans lequel le premier catalyseur a une charge de platine allant d'environ 0,1% en poids à environ 10% en poids, d'environ 0,1% en poids à environ 7,5% en poids, d'environ 0,1% en poids à environ 5% en poids, d'environ 0,1% en poids à environ 4% en poids, d'environ 0,5% en poids à environ 10% en poids, d'environ 0,5% en poids à environ 7,5% en poids, d'environ 0,5% en poids à environ 5% en poids, d'environ 0,5% en poids à environ 4% en poids, d'environ 1% en poids à environ 10% en poids, d'environ 1% en poids à environ 7,5% en poids, d'environ 1% en poids à environ 5% en poids, d'environ 1% en poids à environ 4% en poids, ou d'environ 1% en poids à environ 3% en poids.

11. Procédé de l'une quelconque des revendications précédentes, dans lequel le second catalyseur a une charge d'or allant d'environ 0,1% en poids à environ 10% en poids, d'environ 0,1% en poids à environ 7,5% en poids, d'environ 0,1% en poids à environ 5% en poids, d'environ 0,1% en poids à environ 4% en poids, d'environ 0,1% en poids à environ 3% en poids, d'environ 0,1% en poids à environ 2% en poids, d'environ 0,5% en poids à environ 10% en poids, d'environ 0,5% en poids à environ 7,5% en poids, d'environ 0,5% en poids à environ 5% en poids, d'environ 0,5% en poids à environ 4% en poids, d'environ 0,5% en poids à environ 3% en poids, d'environ 0,5% en poids à environ 2% en poids, d'environ 1% en poids à environ 10% en poids, d'environ 1% en poids à environ 7,5% en poids, d'environ 1% en poids à environ 5% en poids, d'environ 1% en poids à environ 4% en poids, d'environ 1% en poids à environ 3% en poids, ou d'environ 1% en poids à environ 2% en poids.

12. Procédé de l'une quelconque des revendications précédentes, dans lequel la vitesse spatiale horaire de liquide (LHSV) de la zone de réaction est d'environ 0,2 h⁻¹ à environ 50 h⁻¹, d'environ 0,5 h⁻¹ à environ 50 h⁻¹, d'environ 1 h⁻¹ à environ 50 h⁻¹, d'environ 2 h⁻¹ à environ 50 h⁻¹, d'environ 5 h⁻¹ à environ 50 h⁻¹, d'environ 10 h⁻¹ à environ 50 h⁻¹, d'environ 0,2 h⁻¹ à environ 10 h⁻¹, d'environ 0,5 h⁻¹ à environ 10 h⁻¹, d'environ 1 h⁻¹ à environ 10 h⁻¹, d'environ 2 h⁻¹ à environ 10 h⁻¹, ou d'environ 5 h⁻¹ à environ 10 h⁻¹.

13. Procédé de l'une quelconque des revendications précédentes, dans lequel la zone de réaction est chauffée jusqu'à une température allant d'environ 60°C à environ 150°C, d'environ 70°C à environ 150°C, d'environ 80°C à environ 150°C, d'environ 60°C à environ 125°C, d'environ 70°C à environ 125°C, d'environ 80°C à environ 125°C, d'environ 60°C à environ 100°C, d'environ 70°C à environ 100°C, ou d'environ 80°C à environ 100°C.

14. Procédé de l'une quelconque des revendications précédentes, dans lequel la pression partielle d'oxygène est comprise dans la plage allant d'environ 2 psig à environ 2000 psig (d'environ 115,1 kPa à environ 13,89 MPa), d'environ 50 psig à environ 2000 psig (d'environ 446,1 kPa à environ 13,89 MPa), ou d'environ 100 psig à environ 2000 psig (d'environ 790,8 kPa à environ 13,89 MPa).

15. Procédé de l'une quelconque des revendications précédentes, dans lequel l'oxygène est fourni à la zone de réaction sous forme d'air, d'air enrichi en oxygène, d'un mélange comprenant au moins environ 40 ou 50% en volume d'oxygène, d'un mélange comprenant de l'oxygène et de l'azote, ou d'oxygène sensiblement pur.
